# EUROPÄISCHE PATENTSCHRIFT

(11) **EP 3 176 570 B1**
(45) Veröffentlichungstag und Bekanntmachung des Hinweises auf die Patenterteilung: **14.11.2018**
(21) Anmeldenummer: 16200262.0
(22) Anmeldetag: 23.11.2016
(51) Int. Cl.: G01N 24/08, G01R 33/565, G01R 33/44, A61B 90/00, A61B 5/055, G01R 33/28, G01R 33/385

(54) **VERFAHREN ZUR BESTIMMUNG DER POSITION EINES FERROMAGNETISCHEN PARTIKELS UND ZUGEHÖRIGES MRI-SYSTEM**
METHOD FOR DETERMINING THE POSITION OF A FERROMAGNETIC PARTICLE AND ASSOCIATED MRI SYSTEM
PROCÉDÉ DE DÉTERMINATION DE LA POSITION D'UNE PARTICULE FERROMAGNÉTIQUE ET SYSTÈME IRM CORRESPONDANT

(30) Priorität: 02.12.2015 DE 102015224085
(43) Veröffentlichungstag der Anmeldung: 07.06.2017
(73) Patentinhaber: Bruker BioSpin MRI GmbH, 76275 Ettlingen (DE)
(72) Erfinder: NAUERTH, Arno, 76872 Erlenbach (DE)
(74) Vertreter: Kohler Schmid Möbus Patentanwälte

(56) Entgegenhaltungen:
- US-B2- 8 948 841
- YANG H.-J. ET AL.: "Interleaved Magnetic Steering and MR imaging of USPIO Particles in One Dimension: Early Results", INTERNATIONAL SOCIETY FOR MAGNETIC RESONANCE IN MEDICINE, ISMRM, 2030 ADDISON STREET, 7TH FLOOR, BERKELEY, CA 94704 USA, Nr. 3830, 28. April 2014 (2014-04-28), Seite 3830, XP040664858,
- MUNITTA MUTHANA ET AL: "Directing cell therapy to anatomic target sites in vivo with magnetic resonance targeting", NATURE COMMUNICATIONS, Bd. 6, 18. August 2015 (2015-08-18), Seite 8009, XP055276376, United Kingdom ISSN: 2041-1723, DOI: 10.1038/ncomms9009
- JOHANNES RIEGLER ET AL: "Targeted magnetic delivery and tracking of cells using a magnetic resonance imaging system", BIOMATERIALS., Bd. 31, Nr. 20, 10. April 2010 (2010-04-10), Seiten 5366-5371, XP055365933, GB ISSN: 0142-9612, DOI: 10.1016/j.biomaterials.2010.03.032
- Sylvain Martel ET AL: "Interactive System for Medical Interventions Based on Magnetic Resonance Targeting", Achi 2011: The Fourth International Conference on Advances in Computer-Human Interactions, 23. Februar 2011 (2011-02-23), Seiten 197-201, XP055366026, Online Gefunden im Internet: URL:https://www.thinkmind.org/index.php?vi ew=article&articleid=achi_2011_9_20_20127 [gefunden am 2017-04-20]

## Beschreibung

Die Erfindung betrifft ein Verfahren zur Bestimmung der Position wenigstens eines ferromagnetischen Partikels in einer Flüssigkeitsmatrix mittels eines MRI-Systems,
wobei eine MRI-Messsequenz auf ein Messvolumen angewandt wird, in welchem sich das Partikel befindet,
wobei die Messsequenz eine Mehrzahl von Einzelmessungen umfasst, während denen mit dem MRI-System jeweils eine ortskodierende Gradientenschaltung einschließlich eines Anregungsimpulses und einer Signalaufnahme erfolgt,
und wobei mittels der Einzelmessungen Informationen für die Bestimmung der Position des ferromagnetischen Partikels erhalten werden.

Eine solches Verfahren ist bekannt geworden aus Munitta Muthana et al., nature communications, 6:8009, 18. August 2015.

Mikromaschinen und Mikroroboter stellen eine Möglichkeit dar, Manipulationen, Messungen oder andere Funktionen in schwer zugänglichen, insbesondere kleindimensionierten Strukturen mit hoher Präzision vorzunehmen. Die Anwendungsfelder von Mikromaschinen und Mikrorobotern sind vielfältig und liegen derzeit vor allem im Bereich der Produktionstechnik. Jedoch sind auch Anwendungen im Bereich der Biotechnik und der Medizintechnik möglich. Mikrokomponenten können zudem in Verbundmaterialien bzw. Verbundbauteilen die mechanischen Eigenschaften oder andere Materialeigenschaften erweitern oder verbessern.

Bei der Nutzung von Mikromaschinen, Mikrorobotern oder Mikrokomponenten stellt sich allgemein das Problem des Verbringens an einen gewünschten Ort (Einsatzort).

Für Mikroroboter sind eigene Antriebssysteme, etwa Laufbeine, bekannt geworden; solche Antriebssysteme sind jedoch teuer und schwierig zu konstruieren.

Für ferromagnetische Partikel besteht die Möglichkeit, auf diese durch einen Magnetfeldgradienten von außen eine Kraft auszuüben. Dadurch ist es möglich, das ferromagnetische Partikel zu bewegen.

Neben dem Bewegen ist es für die Verbringung an einen gewünschten Ort notwendig, den momentanen Ort des ferromagnetischen Partikels zu überprüfen, insbesondere um gegebenenfalls den momentanen Ort durch weitere Bewegungen zu korrigieren.

Mikromaschinen, Mikroroboter und Mikrokomponenten werden jedoch häufig in Umgebungen eingesetzt, in denen eine unmittelbare optische Beobachtung von außen nicht möglich ist, etwa weil eine Umhüllung oder ein Gehäuse die Beobachtung blockiert, oder auch weil die Mikromaschine, der Mikroroboter oder die Mikrokomponente sich in einer trüben Flüssigkeitsmatrix befindet. Eine solche trübe Flüssigkeitsmatrix kann beispielsweise ein Schmieröl, eine Lösung von unvernetzten oder teilvernetzen Kunststoffbestandteilen oder auch ein Schlicker zur Fertigung einer Keramik sein. In medizinischen Anwendungsfeldern kann die trübe Flüssigkeitsmatrix auch beispielsweise Blut oder Lymphe sein.

Bildgebende Magnetresonanz(=MRI)-Verfahren werden vielfältig genutzt, um Bildinformationen von Strukturen zu erhalten . Dabei ist es möglich, Bildinformationen auch aus dem Inneren einer Struktur zu erhalten, ohne die Struktur zu beschädigen. Beispielsweise werden in klinischen Anwendungen Körperteile von Menschen und Tieren mittels MRI-Verfahren abgebildet.

In der US 7,962,194 B2 ist ein Verfahren und ein System zum Antrieb und zur Kontrolle der Verschiebung eines Mikroroboters in einem Blutgefäß beschrieben. In einer Variante ist vorgesehen, im Wechsel mittels eines MRI-Systems die Position eines ferromagnetischen Körpers in einem Objekt durch eine Bildaufnahmesequenz zu bestimmen und mittels vom MRI-System erzeugten Magnetfeldgradienten den ferromagnetischen Körper in eine gewünschte Richtung auf einen gewünschten Zielort anzutreiben, bis der Körper den gewünschten Zielort erreicht hat. In einem experimentellen Aufbau ist der ferromagnetische Körper in einem Rohr, das durch das MRI-System verlauft, einem Flüssigkeitsstrom ausgesetzt.

Munitta Muthana et al., aaO, beschreiben ein Verfahren zur Verbringung von Makrophagen, die einen onkolytischen Virus tragen, zu Tumoren in Mäusen. Die Makrophagen wurden mit super-paramagnetischen Eisenoxid-Nanopartikeln markiert und wurden mit Gradientenfeldern gesteuert, die mit einem MRI-System erzeugt wurden. Nach diesem Steuern wurden Bilder mittels "RARE" und "gated FLASH"-Sequenzen aufgenommen.

Hsin-Jung Yang et al., Proc. Intl. Soc. Mag. Reson. Med. 22 (2014), 3830, haben vorgeschlagen, in einem MR-Scanner eine zusätzliche Maxwell-Spule anzuordnen, um USIPO-Zellen im Gewebe zu bewegen.

Wenn ein magnetisches Partikel auf dem Weg zu seinem Einsatzort von einer Flüssigkeitsmatrix umgeben ist und durch diese Flüssigkeitsmatrix fortbewegt werden muss, kann mit dem MRI-System jedoch in vielen Fällen die Position des ferromagnetischen Partikels in einer Bildaufnahmesequenz nicht vernünftig bestimmt werden. Das ferromagnetische Partikel erscheint im Bild oftmals unscharf und ist daher in der Bildaufnahme nicht genau lokalisierbar oder gar aus dem Messvolumen verschwunden.

Die US 8,948,841 B2 beschreibt einer Verfahren zum Nachverfolgen eines magnetischen Objekts mittels eines MRI-Systems, wobei mittels Projektionen von magnetischen Isoflächen der Ort des magnetischen Objekts berechnet wird.

Die DE 101 42 253 C1 beschreibt ein Endo-Roboter-System, welches ein magnetisches Grundfeld zur Aufhebung der Gravitation sowie ein dreidimensionales steuerbares Gradientenfeld zur Navigation des Endo-Roboters umfasst. Der Endo-Roboter ist zur Durchführung minimalinvasiver Eingriffe im Körperinneren von Patienten vorgesehen.

### Aufgabe der Erfindung

Es ist die Aufgabe der vorliegenden Erfindung, eine zuverlässigere und präzisere Bestimmung der Position eines ferromagnetischen Partikels in einer Flüssigkeitsmatrix zu ermöglichen.
Weiterhin ist es Aufgabe der Erfindung, ein Verfahren und ein MRI-System vorzuschlagen, die es ermöglichen, das ferromagnetische Partikel in einer Flüssigkeitsmatrix effizient zu positionieren.

### Kurze Beschreibung der Erfindung

Diese Aufgabe wird gelöst durch ein Verfahren der eingangs genannten Art, das dadurch gekennzeichnet ist,
dass die Messsequenz eine Vielzahl von Messblöcken umfasst, die jeweils eine oder mehrere Einzelmessungen und in einer Pause der Ortskodierung einen mit dem MRI-System geschalteten Zwischengradienten umfassen,
wobei die Zwischengradienten so bemessen sind, dass das Partikel im zeitlichen Mittel über jeden Messblock im Wesentlichen an der gleichen Position gehalten wird.

Um genügend Informationen für die Bestimmung der Position des ferromagnetischen Partikels zu erhalten, ist grundsätzlich eine Vielzahl von Einzelmessungen nötig, die in einer Messsequenz nacheinander abgearbeitet werden. Nach den Einzelmessungen wird jeweils eine gewisse Zeit abgewartet, um die Kernspins relaxieren zu lassen.

Während der Einzelmessungen und der Relaxationszeiten wirken Kräfte auf das Partikel, insbesondere durch Gravitation, durch Strömungen in der Flüssigkeitsmatrix oder auch durch die ortskodierenden Gradientenschaltungen während den Einzelmessungen. Diese Kräfte versuchen das Partikel während der Messsequenz zu verschieben. Verschiebungen während der Messsequenz können die Informationen für die Bestimmung der Position des ferromagnetischen Partikels korrumpieren; im schlimmsten Fall wandert das Partikel aus dem Messvolumen heraus.

Im Rahmen der vorliegenden Erfindung ist es vorgesehen, das ferromagnetische Partikel durch Aufschalten von Zwischengradienten zwischen den Einzelmessungen räumlich zu stabilisieren, und so eine Drift des ferromagnetischen Partikels über die Messsequenz zu reduzieren oder aufzuheben. Der Zwischengradient erzeugt eine zusätzliche Kraft auf das Partikel, die typischerweise die übrigen wirkenden, äußeren Kräfte auf das Partikel kompensiert, oder auch die bereits eingetretenen Wirkungen von äußeren Kräften während der vorangegangenen Einzelmessungen wieder rückgängig macht.

Im Rahmen der Erfindung wird eine Messsequenz in eine Vielzahl von Messblöcken unterteilt. Jeder Messblock umfasst einen ersten Teil, während dessen eine oder mehrere Magnetresonanz-Einzelmessungen im Messvolumen stattfinden, und einen zweiten Teil, während dessen ein Zwischengradient geschaltet wird. Für jeden Messblock ergibt sich eine jeweilige mittlere Position des Partikels im Messvolumen. Die Zwischengradienten der Messblöcke werden erfindungsgemäß so bemessen, dass die mittlere Position des Partikels für jeden einzelnen Messblock im Wesentlichen gleich ist. Dadurch ist sichergestellt, dass sich das Partikel bei allen Einzelmessungen einer Messsequenz jeweils im Wesentlichen am gleichen Ort im Messvolumen befindet.

Man beachte, dass eine Drift des ferromagnetischen Partikels innerhalb einer Einzelmessung grundsätzlich nicht vermieden werden kann, da der Zwischengradient die Ortskodierung stören würde und daher nicht während der Ortskodierung appliziert werden kann; zudem kann auch die Ortskodierung selbst eine Drift verursachen. Jedoch kann der Zwischengradient zwischen den Einzelmessungen eine Vergrößerung der Drift verhindern als auch eine bisherige Drift wieder rückgängig machen. Im Einzelfall kann die Drift während einer Einzelmessung so klein sein, dass eine Kompensation nicht nötig ist; in diesem Fall kann sich der Zwischengradient auf ein Halten der Position des Partikels zwischen den Einzelmessungen beschränken.

Bevorzugt wird die Position des Partikels (etwa bezogen auf seinen Mittelpunkt) im zeitlichen Mittel auf den Pixel der Ortskodierung genau gehalten, so dass für jeden Messblock der Messsequenz im zeitlichen Mittel die Position des Partikels in demselben Pixel liegt. In vielen Fällen genügt es aber, wenn die Position des Partikels über die verschiedenen Messblöcke der Messsequenz so weit gehalten wird, dass für eine Drift DRZ des Partikels mit (größtem) Durchmesser PG über die gesamte Messsequenz gilt: DRZ ≤ 5*PG, bevorzugt DRZ ≤ 2*PG, bezogen auf den Abstand von Ausgangspunkt und Endpunkt des Partikels zu Beginn und am Ende der Messsequenz. Zumindest sollte durch die Zwischengradienten sichergestellt werden, dass über die gesamte Messsequenz das Partikel nicht aus dem Messvolumen herausdriftet. Im beispielhaften Falle einer zweidimensionalen Einzelmessung, bei welcher die Scheibendicke des Scheibenselektionsgradienten beispielsweise bei 2mm liegt und der Bildbereich beispielsweise bei 30x30 mm, sollte die Drift des Partikels in Scheibenrichtung nicht mehr als 1mm und im Bildbereich nicht mehr als 15mm in Bezug auf die Bildachsen betragen. Hilfsweise könnte dann das Partikel in benachbarten Scheiben gesucht werden, was allerdings mit einem Mehraufwand verbunden wäre.

Gegenüber einer unkompensierten Drift DRU1 eines Partikels während einer gesamten Messsequenz, zu der es ohne Anwendung von Zwischengradienten kommen würde, kann die Drift DRZ des Partikels während der gesamten Messsequenz unter Anwendung der erfindungsgemäßen Zwischengradienten erheblich verkleinert oder ganz beseitigt werden, beispielsweise mit DRZ ≤ 0,2*DRU1.

Das Verfahren wird mit einem MRI-System durchgeführt, wobei das MRI-System (bzw. dessen Gradientenspulensystem) sowohl dazu eingesetzt wird, im Rahmen der Einzelmessungen ortskodierende Gradientenschaltungen einzurichten, als auch um die Zwischengradienten zu schalten, mit denen die Position des ferromagnetischen Partikels über die Messsequenz stabilisiert wird. Das MRI-System kann daher vorteilhaft doppelt genutzt werden. Das erfindungsgemäße Verfahren ist typischerweise als Betriebsverfahren auf dem MRI-System eingerichtet, wobei eine Steuereinrichtung so eingerichtet bzw. programmiert ist, dass zwischen den Einzelmessungen oder Gruppen von Einzelmessungen der Messsequenz jeweils Zwischengradienten geschaltet werden.

Das ferromagnetische Partikel kann als Mikromaschine, Mikroroboter oder Mikrokomponente ausgebildet sein. Eine typische Größe des ferromagnetischen Partikels liegt unterhalb von einem Millimeter (bezogen auf den größten Durchmesser), in der Regel zwischen 25 µm und 250 µm. Das ferromagnetische Partikel besteht teilweise oder vollständig aus ferromagnetischem Material, insbesondere Eisen, Kobalt oder Nickel oder Legierungen dieser Metalle. Die Flüssigkeitsmatrix ist meist basierend auf Wasser, einem Wasser-Alkohol-Gemisch oder einem organischen Lösungsmittel. Im Falle einer Anwendung aus der Produktionstechnik kann die Flüssigkeitsmatrix ein Öl, insbesondere ein Silikonöl, oder eine wässrige Lösung, insbesondere eine wässrige Tensidlösung, sein. Für eine Anwendung aus der Werkstofftechnik kann die Flüssigkeitsmatrix eine wässrige oder organische Lösung von unvernetzten oder teilvernetzten Kunststoffbestandteilen (insbesondere Monomere und Oligomere für eine Polykondenstation) oder ein Schlicker (Suspension keramischer Partikel) sein. Für die Herstellung eines Verbundwerkstoffs oder Verbundbauteils umspült und/oder durchdringt die Flüssigkeitsmatrix beispielsweise eine Grundstruktur, die mit dem ferromagnetischen Partikel gezielt verstärkt werden soll. Im Falle einer Anwendung in der Biotechnologie oder Medizin kann die Flüssigkeitsmatrix beispielsweise Blut im Blutgefäßsystem oder Lymphe im Lymphgefäßsystem eines Tiers oder eines Menschen sein. Das ferromagnetische Partikel kann im Körper durch die Flüssigkeitsmatrix zu einem Zielort verbracht werden, beispielsweise um dort ein Medikament freizusetzen oder auch um durch Hitzewirkung (etwa infolge induktiven Beheizens des ferromagnetischen Partikels) lokal Körpergewebe zu zerstören. Man beachte, dass solche therapeutischen Behandlungsschritte nicht Teil des beanspruchten Verfahrens sind.

Der Zwischengradient zur Stabilisierung der Partikelposition wird typischerweise in Hinblick auf eine berechnete oder gemessene äußere Kraftwirkung auf das Partikel gezielt gewählt. Im Einzelfall kann auch der Zwischengradient über mehrere vorab stattfindende Messsequenzen bzw. Bildaufnahmen iterativ optimiert werden, bis eine Partikelbewegung minimiert ist, insbesondere ohne die äußere Kraftwirkung auf das Partikel als solche zu berechnen oder zu messen; letzteres Vorgehen kann insbesondere zur Kompensation der im Wesentlichen ortsunabhängigen Gravitationswirkung auf das Partikel genutzt werden. Entsprechendes gilt für einen Fixierungsgradienten (siehe unten).

### Bevorzugte Varianten der Erfindung

In einer bevorzugten Variante des erfindungsgemäßen Verfahrens ist vorgesehen, dass während eines jeweiligen Messblocks das Partikel sich während der einen oder der mehreren Einzelmessungen von einem Ausgangspunkt weg bewegt, und dass der Zwischengradient so bemessen ist, dass während der Wirkung des Zwischengradienten sich das Partikel näherungsweise zurück an den Ausgangspunkt bewegt. Dadurch wird erreicht, dass ein jeder Messblock im Wesentlichen mit der gleichen Position des Partikels beginnen kann.

Vorteilhaft ist eine Variante, bei der sich das Partikel während der einen oder der mehreren Einzelmessungen unter dem Einfluss von Gravitation und/oder einer Strömung der Flüssigkeitsmatrix und/oder einer Wirkung der ortskodierenden Gradientenschaltung bewegt. Diese Einflüsse verursachen besonders häufig eine merkliche Drift des ferromagnetischen Partikels über die Dauer einer Einzelmessung. Durch Kompensation dieser Einflüsse kann in der Regel eine gute Positionsstabilisierung erreicht werden, nach der eine Positionsbestimmung mit hoher Zuverlässigkeit und Genauigkeit möglich ist.

Bevorzugt ist auch eine Variante, bei der die ortskodierende Gradientenschaltung ausbalanciert ist, so dass diese während einer Einzelmessung in Summe keinen Beitrag zu einer Positionsänderung des Partikels leistet. Bei der ausbalancierten Gradientenschaltung erfährt das Partikel durch die Gradientenschaltung typischerweise in einem ersten Teil in eine erste Richtung eine erste Kraft, und in einem zweiten Teil gleicher Dauer in eine zweite, der ersten entgegengesetzte Richtung eine betragsgleiche zweite Kraft. Über die gesamte Einzelmessung heben sich die Beiträge dieser Kräfte auf den Ort des ferromagnetischen Partikels auf. In dieser Variante braucht die ortskodierende Gradientenschaltung für die Positionsstabilisierung des ferromagnetischen Partikels nicht berücksichtigt zu werden, was eine korrekte Einstellung des Zwischengradienten erleichtert. Aufgrund der Viskosität der Flüssigkeitsmatrix wird im Allgemeinen das Partikel nach dem ersten Teil rasch wieder abgebremst, also die Geschwindigkeitsänderung des ersten Teils wieder aufgehoben, bevor der zweite Teil beginnt, so dass im Allgemeinen durch den ersten Teil und den zweiten Teil unabhängig voneinander, nacheinander Teil-Positionsänderungen bewirkt werden, die gegengleich sind.

In einer alternativen Variante ist vorgesehen, dass die ortskodierende Gradientenschaltung nicht ausbalanciert ist, so dass diese einen Beitrag zu einer Positionsänderung des Partikels während einer Einzelmessung leistet, und dass die Zwischengradienten die Beiträge der ortskodierenden Gradientenschaltung mitkompensieren. In diesem Fall kann die ortskodierende Gradientenschaltung frei gewählt werden. Der Zwischengradient wird in Stärke und Dauer so gewählt, dass er auch den Beitrag der ortskodierenden Gradientenschaltung mitberücksichtigt, und insbesondere eine entsprechende Verschiebung des Partikels rückgängig macht.

Vorteilhaft ist weiterhin eine Variante, die vorsieht, dass vor der Messsequenz, insbesondere vor dem Einbringen des Partikels in die Flüssigkeitsmatrix, eine Vermessung von Strömungen in der Flüssigkeitsmatrix erfolgt, mit der ein Beitrag der Strömungen zu einer Positionsänderung des Partikels während einer Einzelmessung und/oder während einer Pause der Ortskodierung ermittelbar ist, und dass die Zwischengradienten die Beiträge der Strömungen mitkompensieren. Durch die Bestimmung der Strömungsverhältnisse vorab kann ein geeigneter, kompensierender Zwischengradient bzw. Beitrag hierfür vorab berechnet und dann während der Messsequenz angewandt werden. Für die freie Bewegung des Partikels in der Flüssigkeitsströmung kann im Allgemeinen in guter Näherung angenommen werden, dass sich das Partikel mit der Geschwindigkeit der Flüssigkeitsströmung bewegt. Zum Bewegen des Partikels entgegen der Flüssigkeitsströmung kann die erforderliche Kraft, abhängig von der Relativgeschwindigkeit, in guter Näherung bestimmt werden (beispielsweise kann in vielen Fällen für kleine, runde Partikel die Kraft über die Gleichung von Stokes bestimmt werden); diese Kraft kann dann über den Magnetfeldgradienten unter Berücksichtigung der magnetischen Permeabilität des Partikelmaterials für die erforderliche Zeit aufgebracht werden. Dadurch kann eine besonders hohe Genauigkeit der Positionsstabilisierung erreicht werden. Man beachte, dass auch die Gravitationseinflüsse typischerweise vorab berechnet werden und ein geeigneter Zwischengradient bzw. Beitrag hierfür während der Messsequenz angewandt wird. Die Gravitationskraft kann beispielsweise für Partikel aus einheitlichem Material aus dem Produkt des Volumens des Partikels, der Dichtedifferenz zwischen Partikelmaterial und Flüssigkeitsmatrix und der Fallbeschleunigung (Ortsfaktor g) (ca. 9,8 N/kg) leicht bestimmt werden.

Bevorzugt ist auch eine Variante, bei der der Zwischengradient zumindest zeitweise das Partikel an eine Randstruktur, an die die Flüssigkeitsmatrix angrenzt, andrückt. Durch das Andrücken wird eine weitere Drift des Partikels verhindert. Wenn eine Drift des Partikels während einer Einzelmessung unmerklich ist, jedoch eine merkliche Drift während der Relaxation zwischen Einzelmessungen zu befürchten ist, kann allein durch Andrücken des Partikels während der Wirkung des Zwischengradienten zwischen den Einzelmessungen eine gute Positionsstabilisierung erreicht werden. Man beachte, dass auch eine Kombination von Zurückschieben des Partikels und Andrücken des Partikels an eine Randstruktur während der Dauer des Zwischengradienten möglich ist.

Beim Andrücken sollte die effektive Kraft auf das Partikel, als Summe der Wirkung des Zwischengradienten, der Gravitation, und ggf. einer Strömung, näherungsweise senkrecht zur Oberfläche der Randstruktur sein. Die Randstruktur sollte so fest sein, dass durch das Andrücken des Partikels die Randstruktur nicht beschädigt oder gar durchstoßen wird. Die Randstruktur wird typischerweise gebildet durch Wände von Kanälen oder Behältern (auch der Kanalboden oder Behälterboden), in denen die Flüssigkeitsmatrix angeordnet ist. In der Flüssigkeitsmatrix kann auch beispielsweise ein poröser Festkörper angeordnet sein, an dem der Partikel an einem bestimmten Ort angeordnet werden soll; der poröse Festkörper bildet dann eine Randstruktur aus. Auch können Polymerfäden oder Polymergespinste in der Flüssigkeitsmatrix eine geeignete Randstruktur ausbilden. Bei medizinischen Anwendungen kann die Randstruktur beispielsweise die Wand eines Blutgefäßes oder Lymphgefäßes eines Tieres oder Patienten sein; das Andrücken hat dabei keine therapeutische Wirkung.

Besonders bevorzugt ist eine Variante, bei der je Messblock lediglich eine Einzelmessung durchgeführt wird. In diesem Fall kann der Zwischengradient zwischen allen Einzelmessungen eingesetzt werden, wodurch eine besonders gute Positionsstabilisierung ermöglicht wird. Die Zwischengradienten können weiterhin während eines besonders großen Anteils der Dauer der Messsequenz eingesetzt werden, um die Drift des Partikels zu minimieren. Alternativ kann beispielsweise ein Zwischengradient nur jeweils nach zwei (oder noch mehr) Einzelmessungen angewandt werden, etwa um seltener die Gradientenspulen schalten zu müssen.

Bei einer bevorzugten Variante wird aus den Ergebnissen der Einzelmessungen der Messsequenz ein vollständiges Bild des Messvolumens erzeugt. In diesem Fall kann nach jeder Messsequenz ein (zweidimensionales oder dreidimensionales) Bild des Messvolumens betrachtet werden, in dem das Partikel ersehen (oder auch markiert) werden kann. Dies ermöglicht ein intuitives, schnelles Erfassen der momentanen Position des Partikels, und etwaige Veränderungen von übrigen Strukturen im Messvolumen können leicht bemerkt werden.

In einer alternativen, vorteilhaften Variante ist vorgesehen,
dass vor der Messsequenz eine Vielzahl von Referenz-Projektionen des Messvolumens ohne das Partikel erstellt wird, insbesondere durch direkte Aufnahme der Referenz-Projektionen des Messvolumens ohne das Partikel oder durch Berechnung aus einer vollständigen Bildaufnahme des Messvolumens ohne das Partikel,
dass mittels der Einzelmessungen eine Vielzahl von Projektionen des Messvolumens mit dem Partikel aufgenommen wird,
und dass durch Vergleich der aufgenommenen Projektionen mit den Referenz-Projektionen die Position des Partikels ermittelt wird. Durch dieses Vorgehen kann die Messsequenz gegenüber einer vollständigen Bildaufnahme verkürzt werden; die Positionsbestimmung kann schneller erfolgen. Bevorzugt wird die über die Projektionen ermittelte Partikelposition in ein vollständiges Bild des Messvolumens eingezeichnet, um auch in diesem Fall eine intuitive Positionserfassung zu ermöglichen.

### Verfahren zum Positionieren

In den Rahmen der vorliegenden Erfindung fällt auch ein Verfahren zum Positionieren wenigstens eines ferromagnetischen Partikels in einer Flüssigkeitsmatrix mittels eines MRI-Systems,
mit folgenden Schritten:
1) die Position des Partikels wird mit dem MRI-System bestimmt;
2) mit dem MRI-System wird ein Positionierungsgradient geschaltet, mit dem die Position des Partikels verändert wird,
das dadurch gekennzeichnet ist,
dass im Rahmen von Schritt 1) ein oben beschriebenes, erfindungsgemäßes Verfahren zur Bestimmung der Position durchgeführt wird,
wobei nach dem Ende der Messsequenz in Schritt 1) bis zum Beginn des Aufschaltens des Positionierungsgradienten in Schritt 2) ein Fixierungsgradient mittels des MRI-System geschaltet wird, mit dem die Position des Partikels im Wesentlichen konstant gehalten wird.

Im Rahmen dieses Verfahrens wird in der Auswertephase nach der Beendigung der Messsequenz, während aus den gewonnenen Rohdaten der Einzelmessungen die eigentliche Positionsinformation über den Partikel im Messvolumen gewonnen wird, und in der Entscheidungsphase (auch genannt Analyse), wenn anhand der eigentlichen Positionsinformationen eine nächste Iteration der Positionsveränderung ermittelt und eingestellt wird, die Position des Partikels mittels eines Fixierungsgradienten fixiert. Dadurch ist sichergestellt, dass zu Beginn der anstehenden Iteration der Positionsveränderung in Schritt 2) sich das Partikel auch noch dort befindet, wo man es aufgrund der vorangegangenen Positionsbestimmung in Schritt 1) auch erwartet. Das Partikel kann insbesondere nicht während der Auswertephase und der Entscheidungsphase aus dem Messvolumen wandern und somit verloren gehen. Für die Auswertephase und Entscheidungsphase steht praktisch beliebig viel Zeit zur Verfügung, ohne dass auf eine Drift des Partikels Rücksicht genommen werden müsste.

Für den Fixierungsgradienten wird wiederum das Gradientenspulensystem des MRI-Systems genutzt, wodurch dieses (neben der Funktionalitäten bei der ortskodierenden Gradientenschaltung der Einzelmessungen und den Zwischengradienten) eine dritte Funktionalität erlangt. Für das Verschieben des Partikels im Rahmen von Schritt 2) wird ebenfalls das Gradientenspulensystem des MRI-Systems genutzt, wodurch dieses auch noch eine vierte Funktionalität erlangt. Eine Steuereinrichtung des MRI-Systems wird entsprechend eingerichtet bzw. programmiert; insofern stellt das erfindungsgemäße Verfahren auch ein Betriebsverfahren für das MRI-System dar.

Bevorzugt wird während der Wirkungsdauer des Fixierungsgradienten die Position des Partikels (etwa bezogen auf seinen Mittelpunkt) im zeitlichen Mittel auf den Pixel der Ortskodierung genau gehalten. In vielen Fällen genügt es aber, wenn die Position des Partikels während der Wirkungsdauer des Fixierungsgradienten zumindest so weit gehalten wird, dass für eine Drift DRF des Partikels mit (größtem) Partikeldurchmesser PG über die Wirkungsdauer gilt, dass DRF≤5*PG, bevorzugt mit DRF≤2*PG, bezogen auf den Abstand von Ausgangspunkt und Endpunkt des Partikels zu Beginn und am Ende der Messsequenz. Zumindest sollte während der Wirkungsdauer des Fixiergradienten das Partikel im Messvolumen gehalten werden. Gegenüber einer unkompensierten Drift DRU2 eines Partikels, zu der es zwischen dem Ende der Messsequenz bis zum Beginn des Aufschaltens des Positionierungsgradienten ohne Anwendung des Fixiergradienten kommen würde, kann die Drift DRF des Partikels während der gleichen Zeit unter Anwendung des erfindungsgemäßen Fixiergradienten erheblich verkleinert oder ganz beseitigt werden, beispielsweise mit DRF ≤ 0,2*DRU2.

Bei einer bevorzugten Variante dieses Verfahrens werden die Schritte 1) und 2) mehrfach wiederholt. Dadurch kann schrittweise (iterativ) ein Partikel sehr genau an einen gewünschten Ort verbracht werden.

Vorteilhaft ist eine Variante, bei der unter der Wirkung des Fixierungsgradienten das Partikel eine Kraft erfährt, die gegengleich ist zur Wirkung der Gravitation und ggf. einer Strömung der Flüssigkeitsmatrix. Entsprechend kann ein in der Flüssigkeitsmatrix befindlicher Partikel während der Wirkung des Fixierungsgradienten schwebend gehalten werden. Eine Wechselwirkung mit einer Randstruktur ist bei diesem Vorgehen nicht erforderlich, so dass diese Variante auch eingesetzt werden kann, wenn keine geeigneten (insbesondere nahegelegenen und robusten) Randstrukturen zur Verfügung stehen.

Ebenfalls vorteilhaft ist eine Variante, bei der der Fixierungsgradient das Partikel an eine Randstruktur, an die die Flüssigkeitsmatrix angrenzt, andrückt. In diesem Fall wird das Partikel durch die Randstruktur mit gehalten. Dieses Vorgehen ist auch möglich, wenn die Wirkung der Gravitation und/oder die Strömungsverhältnisse in der Flüssigkeitsmatrix nicht oder nur ungenau bekannt sind. Bevorzugt wird das Partikel nur mit geringem Abstand zur Randstruktur bewegt (etwa in einem Abstand von bis zu einem Partikeldurchmesser), so dass beim Anlegen an die Randstruktur die Position des Partikels problemlos im Wesentlichen gleich bleiben kann. Typische geeignete Randstrukturen sind poröse Festkörper oder Polymerstränge oder Kanalwände. Bei medizinischen Anwendungen kann die Randstruktur beispielsweise die Wand eines Blutgefäßes oder Lymphgefäßes eines Tieres oder Patienten sein.

Für den Fall, dass das Partikel durch den Fixiergradienten an einer Randstruktur gehalten wird, kann es erforderlich sein, dass der folgende Positionierungsgradient nicht einfach eine lineare Positionsänderung verursacht. Es kann erforderlich sein, dass die Bewegung des Partikels zunächst von der Wandung weg und dann in eine bestimmte Richtung erfolgt, wobei dann auch Richtungsänderungen möglich sind. Das bedeutet, dass der Positionierungsgradient sich in Feldstärke und in Feldrichtung mit der Zeit ändern kann. Somit kann das Partikel auch über eine gekrümmte Positionierungsbahn bewegt werden.

### Erfindungsgemäßes MRI-System

In den Rahmen der vorliegenden Erfindung fällt weiterhin ein MRI-System, umfassend einen Magneten zur Erzeugung eines homogenen Magnetfelds B₀ in einem Messvolumen, ein Gradientenspulensystem zur Erzeugung von ortskodierenden Magnetfeldgradienten im Messvolumen, und ein Hochfrequenz-Anregungs- und Lesespulensystem zum Einstrahlen von Hochfrequenz-Pulsen in das Messvolumen und zum Auslesen des Messvolumens, das dadurch gekennzeichnet ist, dass das MRI-System eingerichtet ist zum Bestimmen der Position eines ferromagnetischen Partikels gemäß einem oben beschriebenen, erfindungsgemäßen Verfahren zur Bestimmung der Position, wobei eine Steuereinrichtung vorhanden ist, die dazu konfiguriert ist, mit dem Gradientenspulensystem Zwischengradienten zwischen Einzelmessungen einer Messsequenz zu schalten, und dass das MRI-System weiterhin eingerichtet ist zum Positionieren des ferromagnetischen Partikels gemäß einem oben beschriebenen, erfindungsgemäßen Verfahren zum Positionieren, wobei die Steuereinrichtung dazu konfiguriert ist, mit dem Gradientenspulensystem Fixierungsgradienten zwischen dem Ende von Messsequenzen und dem Aufschalten von Positionierungsgradienten zu schalten. Das MRI-System bzw. die Steuereinrichtung weist eine entsprechende Programmierung auf, die zwischen Einzelmessungen oder Gruppen von Einzelmessungen einer Messsequenz Zwischengradienten schaltet, und die zwischen Messsequenzen und dem Aufschalten von nachfolgenden Positionierungsgradienten Fixierungsgradienten schaltet. Das erfindungsgemäße MRI-System kann entsprechend verwendet werden in einem der oben beschriebenen, erfindungsgemäßen Verfahren zur Bestimmung der Position und der Positionierung. Mit dem MRI-System ist auf einfache Weise ein genaue Positionskontrolle und Positionskorrektur von ferromagnetischen Partikeln möglich.

Eine bevorzugte Ausführungsform des erfindungsgemäßen MRI-Systems sieht vor, dass das Gradientenspulensystem des MRI-Systems ein erstes Spulensubsystem für die Erzeugung eines Magnetfeldgradienten in einer vertikalen Richtung und zumindest ein zweites Spulensubsystem für die Erzeugung eines Magnetfeldgradienten in einer horizontalen Richtung aufweist, und dass das erste Spulensubsystem eine maximal erzeugbare Gradientenstärke |Gₘₐₓ¹| aufweist, die größer ist als eine maximal erzeugbare Gradientenstärke |Gₘₐₓ²| des zweiten Spulensubsystems,
bevorzugt wobei |Gₘₐₓ¹| ≥ 1,5*|Gₘₐₓ²|. In dieser Ausführungsform wird das erste Spulensubystem besonders stark ausgelegt, um die in vertikaler Richtung wirkende Gravitation kompensieren und wenn nötig überkompensieren zu können. Besonders in niederviskosen Flüssigkeiten kann der Kraftaufwand zur Kompensation der Gravitation insbesondere bei vollmetallischen Teilchen mit herkömmlichen MRI-Systemen erheblich sein und eine vergleichsweise hohe Magnetfeld-Gradientenstärke erfordern. Durch eine verstärktes erstes Spulensubsystem können auch ungünstige Verhältnisse gehandhabt werden. Üblicherweise ist noch ein drittes Spulensubsystem für die Erzeugung eines Magnetfeldgradienten in einer weiteren horizontalen Richtung vorgesehen, wobei die horizontale Richtung und die weitere horizontale Richtung zueinander orthogonal sind; dann ist typischerweise auch |Gₘₐₓ¹| größer als eine maximal erzeugbare Gradientenstärke |Gₘₐₓ³| des dritten Spulensubsystems, bevorzugt wobei |Gₘₐₓ¹| ≥ 1,5*|Gₘₐₓ³|. Das erste Spulensubsystem weist typischerweise eine größere Anzahl von Spulen und/oder eine größere Windungszahl und/oder einen höheren Leiterquerschnitt (für eine höhere Stromtragfähigkeit) als das oder die anderen Spulensubsysteme auf, um die höhere maximale Gradientenstärke zu erreichen.

Vorteilhaft ist weiterhin eine Ausführungsform, bei der das Gradientenspulensystem des MRI-Systems, insbesondere das erste Spulensubsystem, einen Hauptteil und einen Zusatzteil umfasst, wobei die Steuereinrichtung dazu eingerichtet ist, mit dem Hauptteil ortskodierende Gradientenschaltungen, Zwischengradienten, Fixierungsgradienten und/oder Positionierungsgradienten zu schalten, und mit dem Zusatzteil lediglich Zwischengradienten, Fixierungsgradienten und/oder Positionierungsgradienten zu schalten, nicht aber ortskodierende Gradientenschaltungen. Mit dem Zusatzteil kann für die Zwischengradienten , Fixierungsgradienten und Positionierungsgradienten zusätzliche Kraft auf das Partikel zur Verfügung gestellt werden, insbesondere ausreichend zur Kompensation von Gravitation und/oder Strömungen in der Flüssigkeitsmatrix. Der Zusatzteil kann grundsätzlich auch zu dem zweiten und/oder dritten Spulensubsystem hinzugefügt werden. Der Zusatzteil kann vom Hauptteil getrennt angeordnet werden, und kann an einem bestehenden MRI-System auch nachgerüstet werden. In einer ersten Variante werden Zwischengradienten, Fixierungsgradienten und Positionierungsgradienten nur durch den Zusatzteil erzeugt, nicht aber vom Hauptteil; für die ortskodierende Gradientenschaltung wird nur der Hauptteil eingesetzt, nicht aber der Zusatzteil. Dies erlaubt unabhängige Steuerkreise für die Ansteuerung der ortskodierenden Gradientenschaltung einerseits und die Ansteuerung der Zwischengradienten, Fixierungsgradienten und Positionierungsgradienten andererseits; dies ist für eine Nachrüstung eines bestehenden MRI-Systems besonders geeignet. In einer zweiten Variante wird das Zusatzteil zum Festhalten des Partikels in einer Position, also der exakten Kompensation von äußeren Kräften (Haltegradienten; GG, GF, siehe unten) eingesetzt; beispielsweise kann mit dem geschalteten Zusatzteil die Wirkung der Gravitation auf das Partikel kompensiert werden ("Schwebender Partikel"). Davon unabhängig wird dann mit dem Hauptteil eine Bewegung des Partikels gesteuert (GZ, GV, siehe unten). Die Steuerung der Bewegung des Partikels ist dann von der Positionsstabilisierung entkoppelt und entsprechend einfach; insbesondere kann ein Partikel in vertikaler Richtung und horizontaler Richtung gleich effizient bewegt werden. Der Hauptteil wird auch für die ortskodierende Gradientenschaltung eingesetzt, nicht aber der Zusatzteil. Darüber hinaus sind auch weitere Varianten denkbar. Im Allgemeinen übernimmt der Hauptteil zumindest die ortskodierende Gradientenschaltung, und der Zusatzteil nimmt an den Einzelmessungen bzw. der ortskodierenden Gradientenschaltung nicht teil.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

Die Erfindung ist in der Zeichnung dargestellt und wird anhand von Ausführungsbeispielen näher erläutert. Es zeigen:
- Fig. 1: eine schematische Darstellung von angewandten Gradienten während einer Messsequenz im Rahmen der Erfindung, in einer Variante mit ausbalancierter Gradientenschaltung;
- Fig. 2: eine schematische Darstellung von angewandten Gradienten während einer Messsequenz im Rahmen der Erfindung, in einer Variante mit nicht ausbalancierter Gradientenschaltung;
- Fig. 3a: eine schematische Darstellung der Position eines ferromagnetischen Partikels während einer Messsequenz im Rahmen der Erfindung, in einer Variante mit Zurückschieben des Partikels durch den Zwischengradienten;
- Fig. 3b: eine schematische Darstellung der Position eines ferromagnetischen Partikels während einer Messsequenz ähnlich Fig. 3a, jedoch ohne Anwendung eines Zwischengradienten (nicht erfindungsgemäß);
- Fig. 4a: eine schematische Darstellung der Position eines ferromagnetischen Partikels während einer Messsequenz im Rahmen der Erfindung, in einer Variante mit lediglich Halten des Partikels durch den Zwischengradienten;
- Fig. 4b: eine schematische Darstellung der Position eines ferromagnetischen Partikels während einer Messsequenz ähnlich Fig. 4a, jedoch ohne Anwendung eines Zwischengradienten (nicht erfindungsgemäß);
- Fig. 5: ein schematisches Ablaufdiagramm einer Variante des erfindungsgemäßen Verfahrens zur Veränderung der Position eines ferromagnetischen Partikels;
- Fig. 6: eine schematische Darstellung der Position eines ferromagnetischen Partikels während einer Variante des erfindungsgemäßen Verfahrens zur Veränderung der Position des ferromagnetischen Partikels;
- Fig. 7a-7d: schematische Darstellungen eines ferromagnetischen Partikels in einer Flüssigkeitsmatrix in einer umgebenden Randstruktur, während einer Einzelmessung (Fig. 7a), während der Anwendung eines Zwischengradienten (Fig. 7b), während der Anwendung eines Fixierungsgradienten (Fig. 7c) und während der Anwendung eines Positionierungsgradienten (Fig. 7d);
- Fig. 8: eine schematische Darstellung eines erfindungsgemäßen MRI-Systems.

### Überblick über die Erfindung

Die vorliegende Erfindung betrifft ein Verfahren für eine verbesserte Positionsbestimmung eines ferromagnetischen Partikels (Objekts) in einer Flüssigkeitsmatrix, insbesondere viskosen Matrix mit einer Viskosität von 2 mPa*s oder mehr oder auch von 3 mPa*s oder mehr, mit einem Magnetresonanz-Imaging(=MRI)-System. Die vorliegende Erfindung betrifft weiterhin ein Verfahren zum Positionieren und Halten des ferromagnetischen Partikels (Objekts) unter Verwendung der Magnetfeldgradienten des MRI-Systems. Mit Hilfe des MRI-Systems kann das Partikel gleichzeitig sichtbar gemacht werden, sodass man eine visuelle Echtzeit-Kontrolle über das Partikel erhält.

Eine mögliche Anwendung der Erfindung ist es, ferromagnetische Objekte in einem menschlichen oder tierischen Körper mittels der Magnetfeldgradienten zu steuern, um diese an definierte Zielstellen innerhalb des Körpers zu leiten.

Die Gradientenfelder eines MRI-Gerätes werden im Normalfall für die Ortskodierung bei der Bildakquise benötigt und werden außerhalb der Bildaufnahmezeit abgeschaltet. Prinzipiell lässt sich aber mit den Gradientenfeldern auch eine Kraftausübung auf magnetische Objekte erzielen. Dies wird im Rahmen der vorliegenden Erfindung ausgenutzt.

Im Rahmen der Erfindung kann mit Hilfe der Magnetfeldgradienten eine Gegenkraft gegen die auf einem ferromagnetischen Partikel wirkenden Kräfte bereitgestellt werden, welche außerhalb des Messbetriebs eines MRI-Systems am Partikel anliegen, insbesondere aufgrund von Gravitation und Flüssigkeitsströmungen. Insbesondere kann eine vertikale magnetische Gegenkraft mit Hilfe der Gradienten derart eingestellt werden, dass sich das Partikel nicht infolge der Gravitation nach unten bewegt. Weiterhin können die Partikel auch dann stationär gehalten werden, sollten sich diese in einem fließenden Medium befinden. Weiterhin können die Partikel außerhalb der Bildaufnahmezeit kontrolliert bewegt werden. Messabläufe können derart gestaltet werden, dass Gradienten zum Halten und Bewegen der Partikel während der Ortskodierung der Datenaufnahme ("Kodierzeit" der MRI-Messsequenz) ausgeschaltet werden, um die Ortskodierung nicht zu stören, jedoch außerhalb der Ortskodierung der Datenaufnahme die magnetischen Partikel im Mittel stationär gehalten werden oder kontrolliert bewegt werden.

Die Erfindung schlägt insbesondere die Anwendung eines Haltegradienten vor; damit wird allgemein ein Magnetgradient bezeichnet, der außerhalb der Messzeiten angelegt wird, um das Magnetpartikel gegen eine auf das Partikel wirkende Kraft positionsstabil zu halten. Dies kann zum einen ein vertikaler Gradient (vgl. Gegenfeld Gravitation GG) sein, der gegen die Schwerkraft gerichtet ist. Die Stärke dieses Gradienten wird eingestellt in Abhängigkeit von der Masse und der Magnetisierung des Magnetpartikels (beides bekannte Größen). Zum anderen kann der Haltegradient gegen eine Fluidströmung wirken, z.B. wenn sich das Magnetpartikel innerhalb einer sich bewegenden Fluidmatrix befindet (z.B. im Blutstrom). Der Feldvektor des Haltegradienten (vgl. Gegenfeld Fluss GF) ist hier abhängig von der Stärke und Richtung dieses Stroms zu wählen, weshalb im Falle von Strömungen im Allgemeinen zunächst ein Geschwindigkeitsprofil erstellt wird, um den Feldvektor des Gradienten zu bestimmen. Die Aufnahme eines Geschwindigkeitsprofils ist im Stand der Technik bekannt (Literaturbeispiele: Methods Mol. Biol. 2014; 1135:205-11: Quantitative cerebral blood flow measurements using MRI; und weiter Magn. Reson. Med. 2003 Dec; 50(6): 1248-1255: Real-Time Volumetric Flow Measurements With Complex-Difference MRI). Es können auch kombinierte Haltegradienten (GG und GF) gegen die Schwerkraft und das Strömungsprofil eingerichtet werden. Sollten äußere Kräfte, wie z.B. die Gravitation bzw. die Strömung oder auch eine nicht-ausbalancierte ortskodierende Gradientenschaltung, die auf die Partikel wirken, die Partikel während der Kodierzeit merklich aus der Ausgangsposition bewegen, so können die Partikel durch Anlegen eines Kompensationsgradienten wieder zurückbewegt werden.

Die Stärke und Dauer des Kompensationsgradienten ("Fläche") und seine Richtung muss dafür geeignet gewählt werden, insbesondere in Abhängigkeit von Betrag, Richtung und Dauer der zuvor wirkenden äußeren Kräfte.

Die MRI-Messsequenzen werden erfindungsgemäß derart angepasst, dass die magnetischen Objekte im Mittel stationär gehalten werden. Weiterhin ist es möglich, zwischen den Messsequenzen zusätzliche Gradientenfelder anzulegen, um die magnetischen Partikel kontrolliert zu bewegen.

### Erfindungsgemäße Messsequenzen

Im Rahmen der Erfindung wird eine Messsequenz in eine Vielzahl von Messblöcken aufgeteilt. Jeder Messblock umfasst eine Einzelmessung (oder auch mehrere Einzelmessungen) und einen Zwischengradienten.

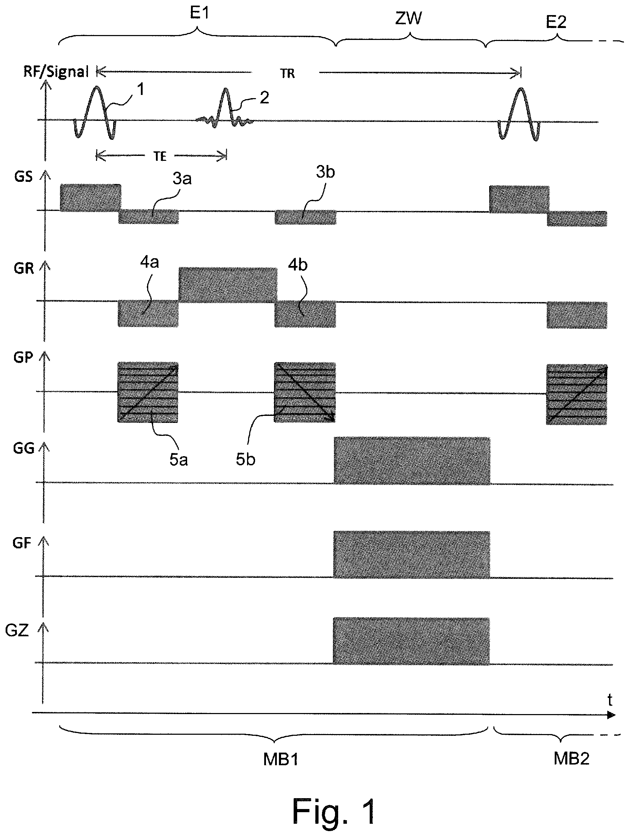

Die **Fig. 1** zeigt einen Ausschnitt einer Messsequenz mit 2D Einzelmessungen in der Gradienten-Echo-(GRE)-Technik; nach rechts ist hierbei die Zeit aufgetragen, nach oben das RF-Signal bzw. der jeweilige geschaltete Gradient. In dem Ausschnitt ist beispielhaft eine erste Einzelmessung E1 und der Beginn einer zweiten Einzelmessung E2 dargestellt; typischerweise verfügt eine Messsequenz über eine Vielzahl weiterer Einzelmessungen. Eine Einzelmessung stellt die Aufnahme eines einzelnen NMR-Signals bei entsprechender Ortskodierung dar.

Die Gesamtheit der erforderlichen Einzelmessungen, um ein vollständiges Bild des Messvolumens oder eine ausreichende Auswahl von Projektionen des Messvolumens zu erstellen, so dass die Position des ferromagnetischen Partikels im Messvolumen ermittelbar ist, bildet eine Messsequenz.

In der gezeigten Variante von Fig. 1 folgt auf jede Einzelmessung jeweils ein so genannter Zwischengradient ZW. Der Zwischengradient ZW wird also in der Messpause zwischen zwei Einzelmessungen E1, E2 angelegt.

Während der Einzelmessung E1 wird ein Hochfrequenz(=RF)-Puls 1 ("Anregung") in das Messvolumen eingestrahlt. Nach der Kodierzeit erfolgt die Aufnahme des RF-Signals 2 ("Antwort") des Messvolumens. Während der Einzelmessung E1 erfolgt die Schaltung der ortskodierenden Gradienten, hier ein Scheibenselektionsgradient GS, ein Lesegradient GR und ein Phasenkodiergradient GP.

Ein zusätzlicher Gradient zum Halten oder Verschieben des ferromagnetischen Partikels würde während der Einzelmessung E1 die Ortskodierung verfälschen, weshalb ein solcher zusätzlicher Gradient nur außerhalb der Kodierzeiten angelegt werden kann; der Zwischengradient ZW wird deshalb hier nur zwischen den Einzelmessungen E1, E2 angelegt. Entsprechend kommt es während der Einzelmessung E1 typischerweise zu einer gewissen Drift des ferromagnetischen Partikels.

Der Zwischengradient ZW umfasst hier als ersten Anteil ein Gegenfeld Gravitation GG, welches genau die (momentane) Wirkung der Gravitation kompensiert. Die aus dem vertikalen Gegenfeld Gravitation GG resultierende Kraft ist der Gravitationskraft entgegengesetzt und so stark, dass das ferromagnetische Partikel (vertikal) schwebend im Messvolumen gehalten wird. Der Zwischengradient ZW umfasst hier auch als zweiten Anteil ein Gegenfeld Fluss GF, das die (momentane) Wirkung einer Flüssigkeitsströmung kompensiert. Die aus dem Gegenfeld Fluss GF resultierende Kraft ist der Kraftwirkung der Flüssigkeitsströmung in der Flüssigkeitsmatrix, der das Partikel ausgesetzt ist, entgegengesetzt und so stark, dass das Partikel durch die Flüssigkeitsströmung nicht fortbewegt wird. Man beachte, dass das Gegenfeld Fluss GF in grundsätzlich jede Raumrichtung gerichtet sein kann, je nachdem, wie die Strömungsverhältnisse sind; die Strömungsverhältnisse sollten für eine möglichst genaue Kompensation entsprechend möglichst genau bekannt sein. Durch das Gegenfeld Gravitation GG und das Gegenfeld Fluss GF allein würde das Partikel zwischen den Einzelmessungen E1 und E2 an konstanter Position gehalten ("Haltegradient"). Weiterhin umfasst der Zwischengradient ZW hier als dritten Anteil ein Gradientenfeld GZ zum Zurückschieben des ferromagnetischen Partikels auf die Position zu Beginn der Einzelmessung E1; dadurch ist sichergestellt, dass die Einzelmessung E1 und die Einzelmessung E2 jeweils mit dem Partikel an derselben Position beginnen, und so alle Einzelmessungen E1, E2 zueinander passen. Durch das Gradientenfeld GZ wird also ein gravitationsbedingtes Absinken und strömungsbedingtes Mitführen des Partikels während der Einzelmessung E1 kompensiert ("Kompensationsgradient") bzw. rückgängig gemacht. In der gezeigten Ausführungsform wirkt das Gradientenfeld GZ während des gesamten Zwischengradienten; alternativ kann das Gradientenfeld GZ auch nur während eines Teils des Zwischengradienten wirken. Die Dauer des Gradientenfelds GZ richtet sich nach der benötigten Zeit, um die Drift aus der Einzelmessung zu kompensieren.

Die erste Einzelmessung E1 und der Zwischengradient ZW bilden einen ersten Messblock MB1 der Messsequenz. Mit der Einzelmessung E2 beginnt ein zweiter Messblock MB2 der Messsequenz. Die Einzelmessung E2 kann dabei erst beginnen, wenn die Wiederholzeit TR abgelaufen ist, welche eine ausreichende Relaxation der Kernspins im Messvolumen sicherstellt.

Bei einer 2D-Gradientenechosequenz folgt dem Anregungspuls (RF-Puls 1) eine dephasierende Gradientenschaltung (vgl. insbesondere Lesegradient GR). Die Rückführung der Phasenverschiebung in Leserichtung und somit die Erzeugung des Gradientenechos wird durch die Umkehrung des Lesegradienten GR erreicht.

Die Variante von Fig. 1 zeigt eine ausbalancierte Gradientenechosequenz, d.h. die Gradienten GS, GR und GP heben sich über die Einzelmessung E1 in ihrer Wirkung auf, sodass sie nach Ende der Einzelmessung E1 keinen Einfluss auf die Position des Partikels haben. Nach dem Einstrahlen des RF-Pulses 1 und nach der Aufnahme des Echosignals, also des RF-Signals 2, sind dazu jeweils einander entsprechende Schaltungsblöcke 3a, 3b und 4a, 4b und 5a, 5b der Gradienten GS, GR und GP vorgesehen.

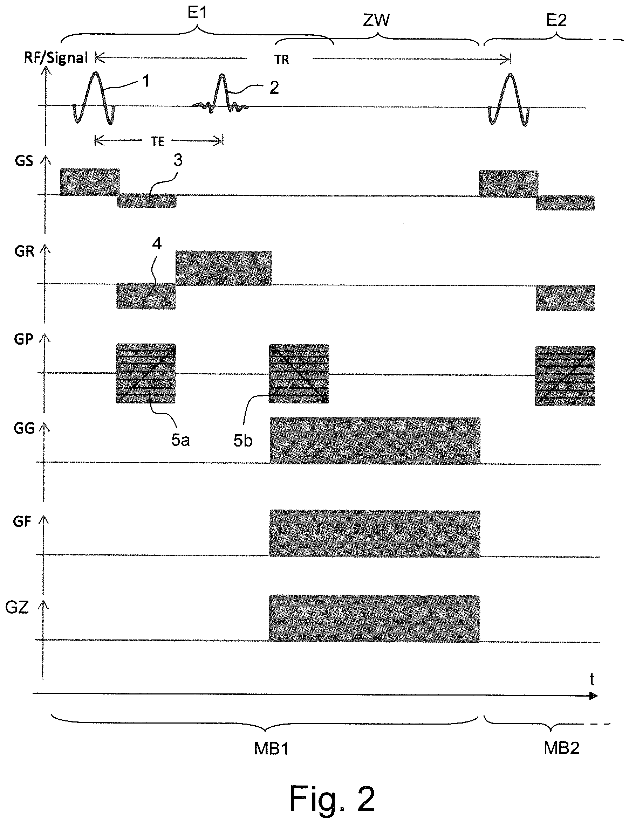

Die Variante von **Fig. 2**, die weitgehend der Variante von Fig. 1 entspricht, zeigt eine nicht ausbalancierte Gradientenechosequenz. Der Lesegradient GR bzw. der Scheibenselektionsgradient GS sind nicht selbst-kompensiert; die Schaltungsblöcke 3, 4 nach dem RF-Puls 1 finden nach der Aufnahme des RF-Signals 2 keine Entsprechung. Die Kompensierung erfolgt hier über den Zwischengradienten ZW, insbesondere über das Gradientenfeld GZ. Dies ist insbesondere vorteilhaft, wenn der Lesegradient GR oder der Scheibenselektionsgradient GS ohnehin in y-Richtung (vertikale Richtung), also der Richtung der Gravitation, stehen. Eine Drift des Partikels durch die nicht ausbalancierte Gradientenechoschaltung während der Einzelmessung E1 wird durch ein entsprechendes Zurückschieben des Partikels mittels des Zwischengradienten ZW bzw. dessen Anteil des Gradientenfelds GZ mit kompensiert. Man beachte, dass in dieser Variante ein geringfügiger Überlapp von Einzelmessung E1 und Zwischengradient ZW vorgesehen ist, nämlich während des zweiten Blocks 5b des Phasenkodiergradienten GP; dieser Überlapp ist unkritisch, da die Aufnahme des RF-Signals 2 bereits abgeschlossen ist. Der Teil 5b des Phasenkodiergradienten dient in diesem Beispiel zur Aufhebung der Kodierung durch den Gradienten 5a und kann gleichzeitig mit dem Zwischengradienten geschaltet werden.

Bei den dargestellten Verfahren wirkt der Zwischengradient ZW, insbesondere mit den Haltegradienten (also das Gegenfeld Gravitation GG und das Gegenfeld Fluss GF), als Spoiler, der die Kernspins dephasiert.

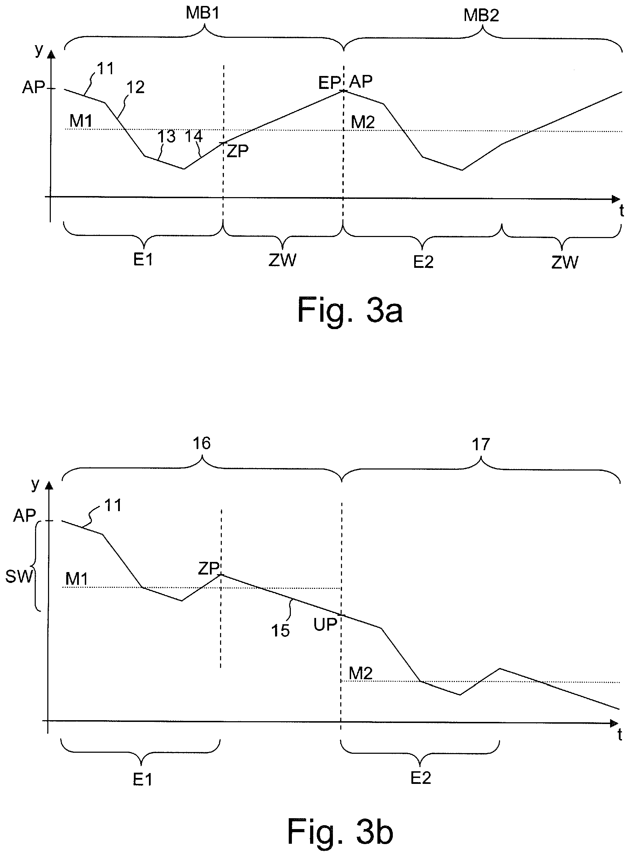

Die **Fig. 3a** erläutert in einem Diagramm die Position eines ferromagnetischen Partikels in vertikaler y-Richtung (nach oben aufgetragen) als Funktion der Zeit t (nach rechts aufgetragen) über einen Ausschnitt einer Messsequenz im Rahmen der Erfindung, mit Zurückschieben des Partikels. Der Ausschnitt umfasst hier beispielhaft zwei Messblöcke MB1, MB2.

Das Partikel sinkt, in Abwesenheit von Magnetfeldgradienten, gravitationsbedingt deutlich (in -y-Richtung) ab, so in den Zeitintervallen 11 und 13 der Einzelmessung E1, insbesondere wenn die Viskosität der Flüssigkeitsmatrix klein und/oder die Masse des Partikels groß ist. Während der Einzelmessung E1 wird zeitweise eine ortskodierende Gradientenschaltung wirksam, die hier über das Zeitintervall 12 das Absinken beschleunigt, und über das Zeitintervall 14 ein leichtes Aufsteigen des Partikels bewirkt ("ausbalancierte Gradientenschaltung"). Die Wirkung der Gravitation verursacht über die Einzelmessung E1 insgesamt ein Absinken des Partikels vom Ausgangspunkt AP zum Zwischenpunkt ZP.

Erfindungsgemäß wird nun während einer Messpause (Pause der Ortskodierung zwischen E1 und E2) ein Zwischengradient ZW angelegt. Dieser zieht das Partikel über die Dauer des Zwischengradienten ZW vom Zwischenpunkt ZP zu einem Endpunkt EP wieder nach oben (in y-Richtung). Der Endpunkt EP ist (zumindest im Wesentlichen) gleich dem Ausgangspunkt AP, d.h. die gesamte Drift in y-Richtung über den Messblock MB1 beträgt idealerweise "null". Entsprechend kann die nächste Einzelmessung E2 mit dem Partikel wieder näherungsweise am selben Ausgangspunkt AP beginnen. Der Verlauf der zweiten Einzelmessung E2 entspricht dann bezüglich der Position des Partikels in y-Richtung über die Zeit t dem Verlauf der ersten Einzelmessung E1.

Über den gesamten ersten Messblock MB1 kann die Position des Partikels (bezüglich der y-Richtung) gemittelt werden. Es ergibt sich die mittlere Position M1. Ebenso kann über den gesamten Messblock MB2 die Position des Partikels gemittelt werden, wobei sich die mittlere Position M2 ergibt. Aufgrund der (im Wesentlichen) gleichen Ausgangspunkte AP zu Beginn der Messblöcke MB1, MB2, die in gleicher Weise ablaufen, sind die mittleren Positionen M1 und M2 (im Wesentlichen) gleich.

Man beachte, dass das Partikel im Rahmen der Erfindung in bis zu drei Dimensionen driften kann und dann entsprechend in bis zu drei Dimensionen mit dem Zwischengradienten kompensiert wird; zur Vereinfachung ist in Fig. 3a nur eine Dimension näher illustriert.

Die Fig. 3b zeigt im Gegensatz dazu einen Ausschnitt aus einer entsprechenden Messsequenz, wobei zwischen den Einzelmessungen E1, E2 kein Zwischengradient geschaltet wird, sondern einfach zugewartet wird (nicht erfindungsgemäß). Nach der Einzelmessung E1 sinkt im Zeitintervall 15 das Partikel gravitationsbedingt frei ab, mit der gleichen Geschwindigkeit wie in Zeitintervall 11. Das Partikel sinkt entsprechend vom Zwischenpunkt ZP zum unkompensierten Punkt UP ab, der um einen Sinkweg SW unterhalb des Ausgangspunkts AP liegt. Von diesem unkompensierten Punkt UP startet die nächste Einzelmessung E2, die analog zur Einzelmessung E1 verläuft. Die mittlere Position M1 des Partikels über das Zeitintervall 16 (vom Beginn der ersten Einzelmessung E1 bis zum Beginn der nächsten Einzelmessung E2) ist dann um den Sinkweg SW höher als die mittlere Position M2 über das gleich lange Zeitintervall 17, das mit der weiteren Einzelmessung E2 beginnt.

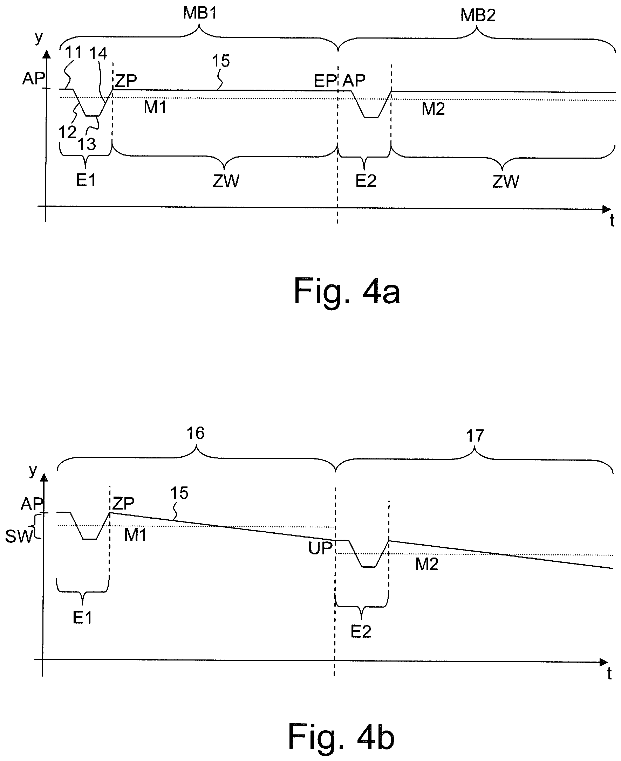

Die **Fig. 4a** erläutert in einem Diagramm die Position eines ferromagnetischen Partikels in vertikaler y-Richtung (nach oben aufgetragen) als Funktion der Zeit t (nach rechts aufgetragen) über einen Ausschnitt einer Messsequenz im Rahmen der Erfindung, mit Festhalten des Partikels. Der Ausschnitt umfasst auch hier beispielhaft zwei Messblöcke MB1, MB2.

In dieser Variante ist die Einzelmessung E1 im Vergleich zum zugehörigen, gesamten Messblock MB1 relativ kurz, die Viskosität der Flüssigkeitsmatrix hoch und/oder die Masse des Partikels klein, so dass es während E1 keine merkliche Positionsänderung durch Gravitation gibt, insbesondere auch nicht in den Zeitintervallen 11, 13. Die Positionsänderungen in den Zeitintervallen 12, 14 aufgrund der ortskodierenden Gradientenschaltung heben sich auf ("ausbalancierte Gradientenschaltung"). Entsprechend ist der Ort des Partikels am Ende der ersten Einzelmessung E1, also der Zwischenpunkt ZP, näherungsweise gleich dem Ausgangspunkt AP.

In der Messpause zwischen erster Einzelmessung E1 und zweiter Einzelmessung E2 wird nun ein Zwischengradient ZW dergestalt angelegt, dass dieser das Partikel genau in Position bezüglich der y-Richtung hält. Dazu kann das Partikel unter Anwendung eines Anlegegradienten (auch als GA bezeichnet) gegen eine nahegelegene Randstruktur gedrückt werden, die die Flüssigkeitsmatrix begrenzt. Alternativ kann auch der angelegte Zwischengradient ZW mit Gegenfeld Gravitation und ggf. Gegenfeld Fluss gerade die äußeren Kräfte auf das Partikel kompensieren. Das Partikel befindet sich am Ende der Wirkungsdauer des Zwischengradienten ZW am Endpunkt EP, der näherungsweise dem Zwischenpunkt ZP und dem Ausgangspunkt AP entspricht.

Entsprechend kann die nächste Einzelmessung E2 mit dem Partikel wieder näherungsweise am selben Ausgangspunkt AP beginnen. Der Verlauf der zweiten Einzelmessung E2 entspricht dann bezüglich der Position des Partikels in y-Richtung über die Zeit t dem Verlauf der ersten Einzelmessung E1. Die mittleren Positionen M1 und M2 im ersten Messblock MB1 und im zweiten Messblock MB2 sind näherungsweise gleich.

Die **Fig. 4b** zeigt im Gegensatz dazu wiederum einen Ausschnitt aus einer entsprechenden Messsequenz, wobei zwischen den Einzelmessungen E1, E2 kein Zwischengradient geschaltet wird, sondern einfach zugewartet wird (nicht erfindungsgemäß). Nach der Einzelmessung E1 sinkt im Zeitintervall 15 das Partikel gravitationsbedingt leicht frei ab. Das Partikel sinkt entsprechend vom Zwischenpunkt ZP zum unkompensierten Punkt UP ab, der um einen Sinkweg SW unterhalb des Ausgangspunkts AP liegt. Von diesem unkompensierten Punkt UP startet die nächste Einzelmessung E2, die analog zur Einzelmessung E1 verläuft. Die mittlere Position M1 des Partikels über das Zeitintervall 16 (vom Beginn der ersten Einzelmessung E1 bis zum Beginn der nächsten Einzelmessung E2) ist dann um den Sinkweg SW höher als die mittlere Position M2 über das gleich lange Zeitintervall 17, das mit der weiteren Einzelmessung E2 beginnt.

### Verschieben des Partikels zwischen Messsequenzen

Die Bestimmung der Position eines Partikels in der Flüssigkeitsmatrix mit einer oben beschriebenen Messsequenz wird in der Regel vorgenommen, um das Partikel in der Flüssigkeitsmatrix bzw. im Messvolumen an einen gewünschten Ort zu positionieren, und dabei nach jeweiligen iterativen Teilschritten des Verschiebens des Partikels die Position zu kontrollieren.

Das MRI-System, das zum Lokalisieren und Positionieren des ferromagnetischen Partikels eingesetzt wird, sollte ein homogenes Magnetfeld (B0-Feld) im Messvolumen erzeugen, so dass das Partikel (ohne Aufschaltung des Gradientenspulensystems) keine ortsverschiebenden Kräfte durch Magnetismus erfährt; eine Ausrichtung des Partikels im B0-Feld ist unvermeidlich.

Grundsätzlich gilt, dass eine vollständige Messsequenz durchgeführt werden muss, um zu der räumlichen Information zu gelangen, die es erlaubt, die Position des Partikels im Messvolumen zu bestimmen.

Wenn das Partikel an eine bestimmte Stelle weiterbefördert werden soll, muss ein Positionierungsgradient geschaltet werden, welcher das Partikel in eine vorgegebene Richtung bewegt. Vorteilhafter Weise wird der Positioniergradient aus einem Haltegradienten (der für sich genommen die Position des Partikels gegen die Schwerkraft und/oder den Flüssigkeitsstrom konstant halten würde) und einem zusätzlichen Verschiebegradienten (der die eigentliche Verschiebung bewirkt, auch als GV bezeichnet) zusammengesetzt, um die Partikelverschiebung einfacher kontrollieren zu können. Die Stärke des Positionierungsgradienten (bzw. des Verschiebegradienten) und die Dauer der Ansteuerung des Positionierungsgradienten (bzw. des Verschiebegradienten) sind der zurückzulegenden Strecke angepasst.

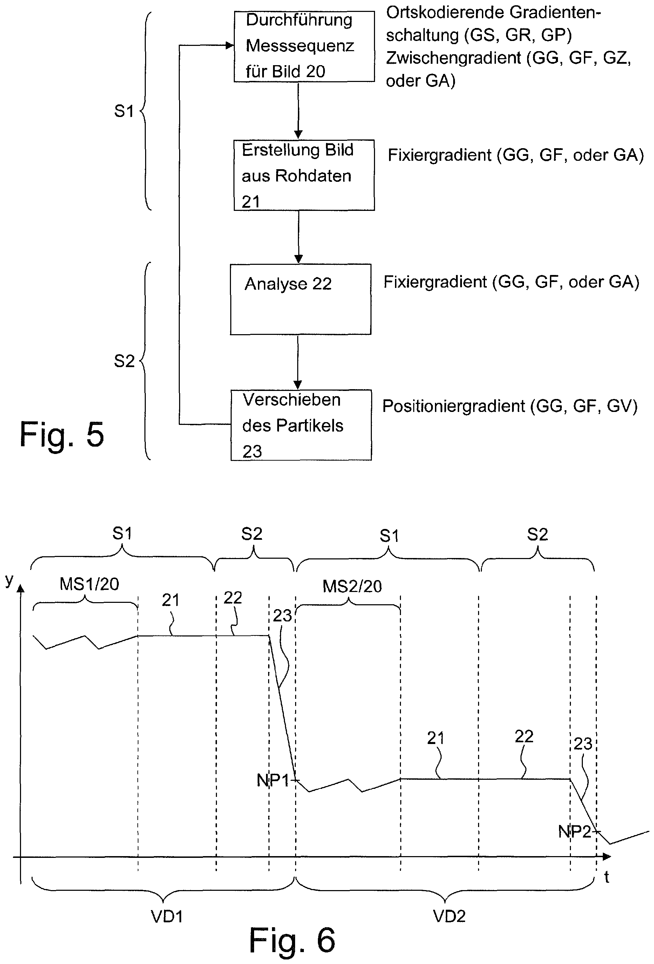

**Fig. 5** zeigt eine schematische Darstellung des Ablaufs einer Variante des erfindungsgemäßen bildgebenden MRI-Verfahrens, mit dem man gleichzeitig ein magnetisches Partikel steuert.

Das Verfahren umfasst in einem ersten Schritt S1 (Bestimmung der Position des Partikels) die Durchführung einer Messsequenz für ein Bild 20 des Messvolumens und die Erstellung des Bildes 21 aus den Rohdaten der Einzelmessungen der Messsequenz, und in einem zweiten Schritt S2 (Veränderung der Position des Partikels) zunächst eine Analyse 22, mit der der nächste Verschiebeschritt geplant und eingestellt wird, und das eigentliche Verschieben des Partikels 23. Anschließend wird die Position des Partikels erneut kontrolliert und verändert und so fort, bis der gewünschte Ort in der Flüssigkeitsmatrix erreicht ist. Auf der rechten Seite der Fig. 5 sind die anzuwendenden Gradientenarten genannt, die man für die entsprechenden Teilschritte 20, 21, 22, 23 schaltet. Der Fixiergradient während der Teilschritte 21, 22 ist dabei entweder als Haltegradient (mit GG, GF, der die Gravitation und ggf. den Flüssigkeitsstrom genau kompensiert) oder als ein Anlegegradient (GA, der das Partikel an einer Randstruktur festhält) ausgebildet.

Die **Fig. 6** illustriert in einem Diagramm das Umpositionieren eines ferromagnetischen Partikels mittels eines MRI-Systems gemäß der Erfindung. Nach oben ist eine Ortskoordinate, hier die vertikale y-Richtung aufgetragen, und nach rechts die Zeit t.

In einem ersten Verfahrensdurchlauf VD1 werden in einer ersten Messsequenz MS1, 20 die Rohdaten für ein Bild des Messvolumens aufgenommen. Innerhalb der einzelnen Messblöcke der Messsequenz MS1 schwankt dabei die Position des Partikels; die mittlere Position des Partikels ist aber für jeden der Messblöcke gleich. Während der Erstellung des Bildes 21 aus den Rohdaten der Einzelmessungen bleibt die Position des Partikels unter Wirkung des Fixiergradienten gleich. Für die nachfolgende Analyse 22 ist nunmehr der momentane Aufenthaltsort des Partikels bekannt, so dass eine nächste Verschiebung (Annäherung an den Zielort) geplant werden kann. Während der Analyse 22 bleibt die Position des Partikels unter weiterer Wirkung des Fixiergradienten gleich. Während der nachfolgenden Verschiebung des Partikels 23 wird dieser an eine neue Position NP1 gebracht. Diese wird im nun folgenden zweiten Verfahrensdurchgang VD2 überprüft, und eine weitere Verschiebung des Partikels zur neuen Position NP2 wird durchgeführt. Daran schließt sich ein weiterer Verfahrensdurchgang an und so fort, bis der gewünschte Zielort erreicht ist.

Man beachte, dass das Partikel im Rahmen der Erfindung in bis zu drei Dimensionen verschoben wird; zur Vereinfachung ist in Fig. 6 nur eine Dimension näher illustriert.

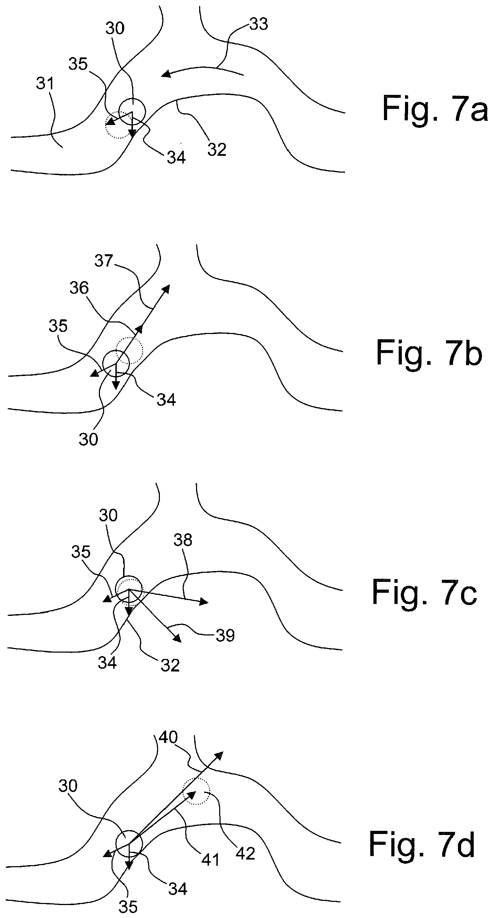

Fig. 7a-7d illustrieren beispielhaft die Kräfteverhältnisse an einem Partikel und dessen Bewegung in einer Flüssigkeitsmatrix mit Randstruktur während verschiedener Verfahrensstadien gemäß der Erfindung.

Wie aus **Fig. 7a** ersichtlich ist, ist ein hier näherungsweise kugelförmiges ferromagnetisches Partikel 30 in einer Flüssigkeitsmatrix 31 angeordnet. Die Flüssigkeitsmatrix 31 wird von einer Randstruktur (hier Wänden von Kanälen) eingeschlossen. Die Flüssigkeitsmatrix 31 unterliegt einer Strömung 33.

Das Partikel 30 wird von der Gravitation 34 nach unten gezogen, und von der Strömungswirkung 35 im Wesentlichen entlang dem Kanalverlauf vorangetrieben. Während einer Einzelmessung (die hier für sich die Position wegen einer ausbalancierten Gradientenschaltung nicht verändert) führen diese äußeren Kräfte zu einer Verschiebung des Partikels 30 an die gepunktet gezeichnete Position.

Deshalb wird erfindungsgemäß nach der Einzelmessung ein Zwischengradient geschaltet, vgl. **Fig. 7b****.** Ein erster Anteil 36 von dessen Wirkung hebt die Gravitation 34 und die Strömungswirkung 35 während seiner Wirkungsdauer auf ("Haltegradient"). Ein weiterer Anteil 37 von dessen Wirkung zieht das Partikel 30 zurück an die vorherige Position zu Beginn der Einzelmessung.

Einzelmessung und Zwischengradient wechseln sich während einer Messsequenz viele Male ab.

Nach Abschluss der Messsequenz soll das Partikel 30 ortsfest gehalten werden, vgl. **Fig. 7c**, etwa für eine Bildberechnung und Analyse für das weitere Vorgehen. In der gezeigten Variante wird hierfür ein Fixiergradient geschaltet, der hier als Anlegegradient gewählt ist. Dessen Kraftwirkung 38 wird teilweise von der Gravitationswirkung 34 und der Strömungswirkung 35 aufgehoben; die resultierende (verbleibende) Kraft 39 zieht das Partikel 30 näherungsweise senkrecht an die lokale Oberfläche des nächstgelegenen Teils der Randstruktur 32. Man beachte, dass für die Einstellung des Fixiergradienten die Gravitationswirkung 34 bekannt sein muss (aus Volumen des Partikels, Dichte des Partikelmaterials sowie der Dichte der Flüssigkeitsmatrix für eine Auftriebsberücksichtigung einfach zu bestimmen), weiterhin die Strömungswirkung bekannt sein muss (aus Größe des Partikels, Viskosität der Flüssigkeitsmatrix und dem Strömungsprofil einfach zu bestimmen; das Strömungsprofil kann über MRI ohne das Partikel vorab bestimmt werden), und weiterhin die Lage der Randstruktur bekannt sein muss (aus einer vorangegangenen MRI-Bildaufnahme ermittelbar).

Nach der Analyse des weiteren Vorgehens kann nunmehr das Partikel 30 verschoben werden, vgl. **Fig. 7d****.** Dafür wird ein Positioniergradient angelegt. Dessen Kraftwirkung 40 dient teilweise der Kompensation der Gravitation 34 und der Strömungswirkung 35 (Anteil "Haltegradient"). Die übrige Kraftwirkung 41 (Anteil "Verschiebegradient") verschiebt das Partikel an eine neue Position 42.

Sodann kann eine weitere Positionsbestimmung und Positionsverschiebung vorgenommen werden, und so fort.

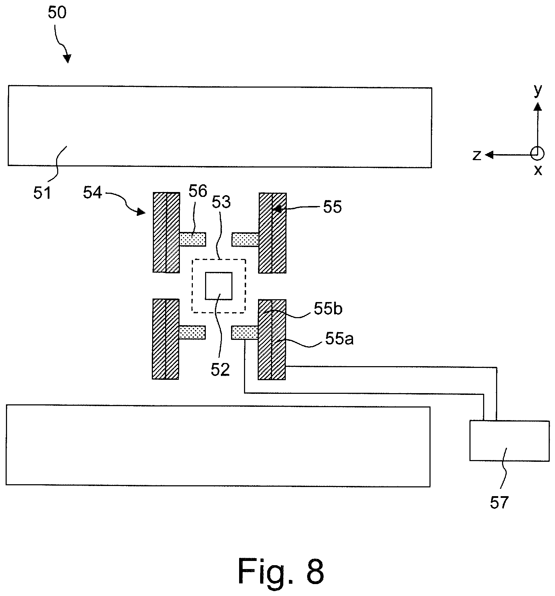

**Fig. 8** zeigt eine Ausführungsform eines erfindungsgemäßen MRI-Systems 50, insbesondere zur Durchführung einer Positionsbestimmung und/oder Positionsänderung eines ferromagnetischen Partikels (Objekts) im Rahmen der Erfindung.

Das MRI-System 50 weist einen Magneten (Hauptmagnetspulensystem, typischerweise supraleitend) 51 auf, mit dem in einem Messvolumen 52 ein starkes, homogenes Magnetfeld B0 in horizontale z-Richtung erzeugt werden kann, typischerweise von einer Stärke von 1 T oder mehr. Mit einem Hochfrequenz-Anregungs- und Lesespulensystem 53 können RF-Pulse in das Messvolumen 52 eingestrahlt werden und ein RF-Signal des Messvolumens ausgelesen werden. Weiterhin ist ein Gradientenspulensystem 54 vorgesehen, mit dem Magnetfeldgradienten im Messvolumen 52 erzeugt werden können.

Das Gradientenspulensystem 54 umfasst hier ein erstes Spulensubsystem 55, mit dem Magnetfeldgradienten in einer vertikalen y-Richtung erzeugt werden können, d.h. die Magnetfeldstärke ändert sich entlang y-Richtung. Weiterhin ist ein zweites Spulensubsystem 56 vorgesehen, mit dem Magnetfeldgradienten in der horizontalen z-Richtung erzeugt werden können, d.h. die Magnetfeldstärke ändert sich entlang der z-Richtung.

Das erste Spulensubsystem 55 ist hier deutlich stärker ausgebildet als das zweite Spulensubsystem 56, beispielsweise für eine wenigstens 1,5 mal größere Gradientenstärke, so dass auch eine erhebliche Graviationskraft in y-Richtung auf ein ferromagnetisches Partikel problemlos kompensiert werden kann. Das erste Spulensubsystem 55 ist dabei mit einem Hauptteil 55a und einem hier innenliegenden Zusatzteil 55b ausgestaltet. Der Zusatzteil 55b wird lediglich eingesetzt, wenn zumindest auch eine Gravitationswirkung kompensiert werden soll (also bei Zwischengradienten, Fixierungsgradienten und Positionierungsgradienten), nicht aber für eine ortskodierende Gradientenschaltung. Der Hauptteil wird dazu eingesetzt, ortskodierende Gradientenschaltungen vorzunehmen; er kann weiterhin mit eingesetzt werden, um eine Gravitationswirkung zu kompensieren (also für Zwischengradienten, Fixierungsgradienten und Positionierungsgradienten).

Das Gradientenspulensystem 54 wird über eine elektronische Steuereinrichtung 57 gesteuert, die programmiert ist, zwischen Einzelmessungen oder Gruppen von Einzelmessungen einer Messsequenz Zwischengradienten zu schalten, und zwischen Messsequenzen Fixiergradienten und Positioniergradienten zu schalten.

### Bezuqszeichenliste

- 1: RF-Puls (Anregung)
- 2: RF-Signal (Antwort)
- 3: unkompensierter Schaltungsblock (GS)
- 3a, 3b: kompensierte Schaltungsblöcke (GS)
- 4: unkompensierter Schaltungsblock (GR)
- 4a, 4b: kompensierte Schaltungsblöcke (GR)
- 5a, 5b: kompensierte Schaltungsblöcke (GP)
- 11-14: Zeitintervalle während Einzelmessung
- 15: Zeitintervall nach Einzelmessung
- 16, 17: Zeitintervalle von Beginn Einzelmessung bis Beginn nächste Einzelmessung
- 20: Durchführung Messsequenz für Bild
- 21: Erstellung Bild aus Rohdaten
- 22: Analyse
- 23: Verschieben des Partikels
- 30: ferromagnetisches Partikel (Objekt)
- 31: Flüssigkeitsmatrix
- 32: Randstruktur
- 33: Strömung
- 34: Gravitation (Kraft)
- 35: Strömungswirkung (Kraft)
- 36: erster Anteil Wirkung Zwischengradient (Kraft)
- 37: zweiter Anteil Wirkung Zwischengradient (Kraft)
- 38: Wirkung Anlegegradient (Kraft)
- 39: resultierende Kraft
- 40: Kraftwirkung Positioniergradient (Kraft)
- 41: übrige Kraftwirkung (Kraft)
- 42: neue Position des Partikels
- 50: MRI-System
- 51: Magnet
- 52: Messvolumen
- 53: Hochfrequenz-Anregungs- und Lesespulensystem
- 54: Gradientenspulensystem
- 55: erstes Spulensubsystem
- 55a: Hauptteil
- 55b: Zusatzteil
- 56: zweites Spulensubsystem
- 57: Steuereinrichtung

- AP: Ausgangspunkt
- E1: erste Einzelmessung
- E2: zweite Einzelmessung
- EP: Endpunkt
- GA: Anlegegradient
- GF: Gegenfeld Fluss (Gradient)
- GG: Gegenfeld Gravitation (Gradient)
- GP: Phasenkodiergradient
- GR: Lesegradient
- GS: Scheibenselektionsgradient
- GV: Verschiebegradient
- GZ: Gradientenfeld zum Zurückschieben des ferromagnetischen Partikels (Gradient)
- M1: mittlere Position während erstem Messblock
- M2: mittlere Position während zweitem Messblock
- MB1: erster Messblock
- MB2: zweiter Messblock
- MS1: erste Messsequenz
- MS2: zweite Messsequenz
- NP1: erste neue Position
- NP2: zweite neue Position
- SW: Sinkweg
- S1: Schritt 1
- S2: Schritt 2
- t: Zeit
- TE: Echozeit
- UP: unkompensierter Punkt
- x: horizontale Richtung
- y: vertikale Richtung
- VD1: erster Verfahrensdurchgang
- VD2: zweiter Verfahrensdurchgang
- z: horizontale Richtung
- ZP: Zwischenpunkt
- ZW: Zwischengradient

## Patentansprüche

1. Verfahren zur Bestimmung der Position wenigstens eines ferromagnetischen Partikels (30) in einer Flüssigkeitsmatrix (31) mittels eines MRI-Systems (50), wobei eine MRI-Messsequenz (MS1, MS2) auf ein Messvolumen (52) angewandt wird (20), in welchem sich das Partikel (30) befindet,
wobei die Messsequenz (MS1, MS2) eine Mehrzahl von Einzelmessungen (E1, E2) umfasst, während denen mit dem MRI-System (50) jeweils eine ortskodierende Gradientenschaltung einschließlich eines Anregungsimpulses (1) und einer Signalaufnahme (2) erfolgt,
und wobei mittels der Einzelmessungen (E1, E2) Informationen für die Bestimmung der Position des ferromagnetischen Partikels (30) erhalten werden,
**dadurch gekennzeichnet,**
**dass** die Messsequenz (MS1, MS2) eine Vielzahl von Messblöcken (MB1, MB2) umfasst, die jeweils eine oder mehrere Einzelmessungen (E1, E2) und in einer Pause der Ortskodierung einen mit dem MRI-System (50) geschalteten Zwischengradienten (ZW) umfassen,
wobei die Zwischengradienten (ZW) so bemessen sind, dass das Partikel (30) im zeitlichen Mittel über jeden Messblock (MB1, MB2) im Wesentlichen an der gleichen Position (M1, M2) gehalten wird.

2. Verfahren nach Anspruch 1, **dadurch gekennzeichnet, dass** während eines jeweiligen Messblocks (MB1, MB2) das Partikel (30) sich während der einen oder der mehreren Einzelmessungen (E1, E2) von einem Ausgangspunkt (AP) weg bewegt, und dass der Zwischengradient (ZW) so bemessen ist, dass während der Wirkung des Zwischengradienten (ZW) sich das Partikel (30) näherungsweise zurück an den Ausgangspunkt (AP) bewegt.

3. Verfahren nach Anspruch 2, **dadurch gekennzeichnet, dass** sich das Partikel (30) während der einen oder der mehreren Einzelmessungen (E1, E2) unter dem Einfluss von Gravitation (34) und/oder einer Strömung (35) der Flüssigkeitsmatrix (31) und/oder einer Wirkung der ortskodierenden Gradientenschaltung bewegt.

4. Verfahren nach einem der Ansprüche 1 oder 2, **dadurch gekennzeichnet, dass** die ortskodierende Gradientenschaltung ausbalanciert ist, so dass diese während einer Einzelmessung (E1, E2) in Summe keinen Beitrag zu einer Positionsänderung des Partikels (30) leistet.

5. Verfahren nach einem der Ansprüche 1 bis 3, **dadurch gekennzeichnet, dass** die ortskodierende Gradientenschaltung nicht ausbalanciert ist, so dass diese einen Beitrag zu einer Positionsänderung des Partikels (30) während einer Einzelmessung (E1, E2) leistet, und dass die Zwischengradienten (ZW) die Beiträge der ortskodierenden Gradientenschaltung mitkompensieren.

6. Verfahren nach einem der vorhergehenden Ansprüche, **dadurch gekennzeichnet, dass** vor der Messsequenz (MS1, MS2), insbesondere vor dem Einbringen des Partikels (30) in die Flüssigkeitsmatrix (31), eine Vermessung von Strömungen (33) in der Flüssigkeitsmatrix (31) erfolgt, mit der ein Beitrag der Strömungen (33) zu einer Positionsänderung des Partikels (30) während einer Einzelmessung (E1, E2) und/oder während einer Pause der Ortskodierung ermittelbar ist, und dass die Zwischengradienten (ZW) die Beiträge der Strömungen (33) mitkompensieren.

7. Verfahren nach einem der vorhergehenden Ansprüche, **dadurch gekennzeichnet, dass** der Zwischengradient (ZW) zumindest zeitweise das Partikel (30) an eine Randstruktur (32), an die die Flüssigkeitsmatrix (31) angrenzt, andrückt.

8. Verfahren nach einem der vorhergehenden Ansprüche, **dadurch gekennzeichnet, dass** je Messblock (MB1, MB2) lediglich eine Einzelmessung (E1, E2) durchgeführt wird.

9. Verfahren nach einem der Ansprüche 1 bis 8, **dadurch gekennzeichnet, dass** aus den Ergebnissen der Einzelmessungen (E1, E2) der Messsequenz (MS1, MS2) ein vollständiges Bild des Messvolumens (52) erzeugt wird.

10. Verfahren nach einem der Ansprüche 1 bis 8, **dadurch gekennzeichnet, dass** vor der Messsequenz (MS1, MS2) eine Vielzahl von Referenz-Projektionen des Messvolumens (52) ohne das Partikel (30) erstellt wird, insbesondere durch direkte Aufnahme der Referenz-Projektionen des Messvolumens (52) ohne das Partikel (30) oder durch Berechnung aus einer vollständigen Bildaufnahme des Messvolumens (52) ohne das Partikel (30), dass mittels der Einzelmessungen (E1, E2) eine Vielzahl von Projektionen des Messvolumens (52) mit dem Partikel (30) aufgenommen wird,
und dass durch Vergleich der aufgenommenen Projektionen mit den Referenz-Projektionen die Position des Partikels (30) ermittelt wird.

11. Verfahren zum Positionieren wenigstens eines ferromagnetischen Partikels (30) in einer Flüssigkeitsmatrix (31) mittels eines MRI-Systems (50),
mit folgenden Schritten:
1) die Position des Partikels (30) wird mittels des MRI-Systems (50) bestimmt (S1);
2) mit dem MRI-System (50) wird ein Positionierungsgradient geschaltet (23), mit dem die Position des Partikels (30) verändert wird (S2),
**dadurch gekennzeichnet,**
**dass** im Rahmen von Schritt 1) ein Verfahren gemäß einem der Ansprüche 1 bis 10 durchgeführt wird,
wobei nach dem Ende der Messsequenz (20) in Schritt 1) (S1) bis zum Beginn des Aufschaltens des Positionierungsgradienten (23) in Schritt 2) (S2) ein Fixierungsgradient mittels des MRI-System (50) geschaltet wird, mit dem die Position des Partikels (30) im Wesentlichen konstant gehalten wird.

12. Verfahren nach Anspruch 11, **dadurch gekennzeichnet, dass** die Schritte 1) (S1) und 2) (S2) mehrfach wiederholt werden.

13. Verfahren nach Anspruch 11 oder 12, **dadurch gekennzeichnet, dass** unter der Wirkung des Fixierungsgradienten das Partikel (30) eine Kraft erfährt, die gegengleich ist zur Wirkung der Gravitation (34) und ggf. einer Strömung (35) der Flüssigkeitsmatrix (31).

14. Verfahren nach einem der Ansprüche 11 oder 12 **dadurch gekennzeichnet, dass** der Fixierungsgradient das Partikel (30) an eine Randstruktur (32), an die die Flüssigkeitsmatrix (31) angrenzt, andrückt.

15. MRI-System (50), umfassend einen Magneten (51) zur Erzeugung eines homogenen Magnetfelds B₀ in einem Messvolumen (52), ein Gradientenspulensystem (54) zur Erzeugung von ortskodierenden Magnetfeldgradienten im Messvolumen (52), und ein Hochfrequenz-Anregungs- und Lesespulensystem (53) zum Einstrahlen von Hochfrequenz-Pulsen (1) in das Messvolumen (52) und zum Auslesen des Messvolumens (52),
**dadurch gekennzeichnet,**
**dass** das MRI-System (50) eingerichtet ist zum Bestimmen der Position eines ferromagnetischen Partikels (30) gemäß einem der Ansprüche 1 bis 10, wobei eine Steuereinrichtung (57) vorhanden ist, die dazu konfiguriert ist, mit dem Gradientenspulensystem (54) Zwischengradienten (ZW) zwischen Einzelmessungen (E1, E2) einer Messsequenz (MS1, MS2) zu schalten, und dass das MRI-System (50) weiterhin eingerichtet ist zum Positionieren des ferromagnetischen Partikels (30) gemäß einem der Ansprüche 11 bis 14, wobei die Steuereinrichtung (57) dazu konfiguriert ist, mit dem Gradientenspulensystem (54) Fixierungsgradienten zwischen dem Ende von Messsequenzen (20) und dem Aufschalten von Positionierungsgradienten (23) zu schalten.

16. MRI-System (50) nach Anspruch 15, **dadurch gekennzeichnet,**
**dass** das Gradientenspulensystem (54) des MRI-Systems (50) ein erstes Spulensubsystem (55) für die Erzeugung eines Magnetfeldgradienten in einer vertikalen Richtung (y) und zumindest ein zweites Spulensubsystem (56) für die Erzeugung eines Magnetfeldgradienten in einer horizontalen Richtung (z, x) aufweist,
und **dass** das erste Spulensubsystem (55) eine maximal erzeugbare Gradientenstärke |Gₘₐₓ¹| aufweist, die größer ist als eine maximal erzeugbare Gradientenstärke |Gₘₐₓ²| des zweiten Spulensubsystems (56),
bevorzugt wobei |Gₘₐₓ¹| ≥ 1,5*|Gₘₐₓ²|.

17. MRI-System (50) nach Anspruch 15 oder 16, **dadurch gekennzeichnet, dass** das Gradientenspulensystem (54) des MRI-Systems (50), insbesondere das erste Spulensubsystem (55), einen Hauptteil (55a) und einen Zusatzteil (55b) umfasst, wobei die Steuereinrichtung (57) dazu eingerichtet ist, mit dem Hauptteil (55a) ortskodierende Gradientenschaltungen, Zwischengradienten, Fixierungsgradienten und/oder Positionierungsgradienten zu schalten, und mit dem Zusatzteil (55b) lediglich Zwischengradienten, Fixierungsgradienten und/oder Positionierungsgradienten zu schalten, nicht aber ortskodierende Gradientenschaltungen.

## Claims

1. Method for determining the position of at least one ferromagnetic particle (30) in a liquid matrix (31) by means of an MRI system (50), with an MRI measurement sequence (MS1, MS2) being applied (20) to a measurement volume (52) in which the particle (30) is situated, wherein the measurement sequence (MS1, MS2) comprises a plurality of individual measurements (E1, E2), during each of which there is a spatially encoding gradient switching operation, including an excitation pulse (1) and signal recording (2), by means of the MRI system (50),
and wherein information for determining the position of the ferromagnetic particle (30) is obtained by means of the individual measurements (E1, E2), **characterized in that** the measurement sequence (MS1, MS2) comprises a multiplicity of measurement blocks (MB1, MB2), which each comprise one or more individual measurements (E1, E2) and in a pause of the spatial encoding an intermediate gradient (ZW) switched by means of the MRI system (50), the intermediate gradients (ZW) being dimensioned such that, averaged over time, the particle (30) is kept substantially in the same position (M1, M2) over each measurement block (MB1, MB2).

2. Method according to Claim 1, **characterized in that** the particle (30) moves away from an initial point (AP) during the one or more individual measurements (E1, E2) during a respective measurement block (MB1, MB2) and **in that** the intermediate gradient (ZW) is dimensioned such that, during the action of the intermediate gradient (ZW), the particle (30) approximately moves back to the initial point (AP).

3. Method according to Claim 2, **characterized in that** the particle (30) moves under the influence of gravity (34) and/or a flow (35) of the liquid matrix (31) and/or an action of the spatially encoding gradient switching operation during the one or more individual measurements (E1, E2).

4. Method according to one of Claims 1 or 2, **characterized in that** the spatially encoding gradient switching operation is balanced, and so, in total, does not contribute to a change in position of the particle (30) during an individual measurement (E1, E2).

5. Method according to one of Claims 1 to 3, **characterized in that** the spatially encoding gradient switching operation is unbalanced, and so contributes to a change in position of the particle (30) during an individual measurement (E1, E2), and **in that** the intermediate gradients (ZW) also compensate the contributions of the spatially encoding gradient switching operation.

6. Method according to one of the preceding claims, **characterized in that** flows (33) in the liquid matrix (31) are measured prior to the measurement sequence (MS1, MS2), in particular prior to the introduction of the particle (30) into the liquid matrix (31), said measurement rendering a contribution of the flows (33) to a change in position of the particle (30) during an individual measurement (E1, E2) and/or during a pause of the spatial encoding ascertainable, and **in that** the intermediate gradients (ZW) also compensate the contributions of the flows (33).

7. Method according to one of the preceding claims, **characterized in that** the intermediate gradient (ZW), at least intermittently, presses the particle (30) against an edge structure (32) neighbouring the liquid matrix (31).

8. Method according to one of the preceding claims, **characterized in that** only one individual measurement (E1, E2) is carried out during each measurement block (MB1, MB2).

9. Method according to one of Claims 1 to 8, **characterized in that** a complete image of the measurement volume (52) is generated from the results of the individual measurements (E1, E2) of the measurement sequence (MS1, MS2).

10. Method according to one of Claims 1 to 8, **characterized**
**in that** a multiplicity of reference projections of the measurement volume (52) without the particle (30) are created prior to the measurement sequence (MS1, MS2), in particular by directly recording the reference projections of the measurement volume (52) without the particle (30) or by calculation from a complete image recording of the measurement volume (52) without the particle (30),
**in that** a multiplicity of projections of the measurement volume (52) with the particle (30) are recorded by means of the individual measurements (E1, E2), and in that the position of the particle (30) is ascertained by comparison between the recorded projections and the reference projections.

11. Method for positioning at least one ferromagnetic particle (30) in a liquid matrix (31) by means of an MRI system (50), comprising the following steps:
1) determining (S1) the position of the particle (30) by means of the MRI system (50);
2) switching (23) a positioning gradient with the MRI system (50), by means of which the position of the particle (30) is changed (S2),
**characterized**
**in that** a method according to one of Claims 1 to 10 is performed within the scope of step 1),
with a fixing gradient, by means of which the position of the particle (30) is kept substantially constant, being switched by means of the MRI system (50) after the end of the measurement sequence (20) in step 1) (S1) and until the start of the application of the positioning gradient (23) in step 2) (S2).

12. Method according to Claim 11, **characterized in that** steps 1) (S1) and 2) (S2) are repeated a number of times.

13. Method according to Claim 11 or 12, **characterized in that** the particle (30) experiences a force under the action of the fixing gradient, said force being equal and opposite to the action of gravity (34) and, where necessary, of a flow (35) of the liquid matrix (31).

14. Method according to either of Claims 11 or 12, **characterized in that** the fixing gradient presses the particle (30) against an edge structure (32) neighbouring the liquid matrix (31).

15. MRI system (50), comprising a magnet (51) for generating a homogeneous magnetic field B₀ in a measurement volume (52), a gradient coil system (54) for generating spatially encoding magnetic field gradients in the measurement volume (52) and a radio frequency excitation and readout coil system (53) for radiating radio frequency pulses (1) into the measurement volume (52) and for reading the measurement volume (52),
**characterized**
**in that** the MRI system (50) is configured to determine the position of a ferromagnetic particle (30) according to one of Claims 1 to 10, a control device (57) being present, the latter configured to switch intermediate gradients (ZW) between individual measurements (E1, E2) of a measurement sequence (MS1, MS2) with the gradient coil system (54),
and **in that** the MRI system (50) is further configured to position the ferromagnetic particle (30) according to one of Claims 11 to 14, the control device (57), with the gradient coil system (54), being configured to switch fixing gradients between the end of measurement sequences (20) and the application of positioning gradients (23).

16. MRI system (50) according to Claim 15, **characterized**
**in that** the gradient coil system (54) of the MRI system (50) comprises a first coil subsystem (55) for generating a magnetic field gradient in a vertical direction (y) and at least one second coil subsystem (56) for generating a magnetic field gradient in a horizontal direction (z, x),
and **in that** the first coil subsystem (55) has a maximum generable gradient strength |Gₘₐₓ¹| which is greater than a maximum generable gradient strength |Gₘₐₓ²| of the second coil subsystem (56),
preferably with |Gₘₐₓ¹| ≥ 1.5*|Gₘₐₓ²|.

17. MRI system (50) according to Claim 15 or 16, **characterized in that** the gradient coil system (54) of the MRI system (50), in particular the first coil subsystem (55), comprises a main part (55a) and an additional part (55b), the control device (57) being configured to switch spatially encoding gradient switching operations, intermediate gradients, fixing gradients and/or positioning gradients with the main part (55a), and only to switch intermediate gradients, fixing gradients and/or positioning gradients, but not spatially encoding gradient switching operations, with the additional part (55b).

## Revendications

1. Procédé pour déterminer la position d'au moins une particule ferromagnétique (30) dans une matrice liquide (31) au moyen d'un système d'IRM (50), dans lequel une séquence de mesure IRM (MS1, MS2) est appliquée (20) sur un volume de mesure (52) dans lequel se trouve la particule (30), dans lequel la séquence de mesure (MS1, MS2) comprend une pluralité de mesures individuelles (E1, E2) au cours de chacune desquelles une commutation de gradients à codage spatial, incluant une impulsion d'excitation (1) et une acquisition de signal (2), est effectuée avec le système d'IRM (50),
et dans lequel des informations pour déterminer la position de la particule ferromagnétique (30) sont obtenues au moyen des mesures individuelles (E1, E2), **caractérisé en ce**
**que** la séquence de mesure (MS1, MS2) comprend une pluralité de blocs de mesure (MB1, MB2) qui comprennent chacun une ou plusieurs mesures individuelles (E1, E2) et, dans une pause du codage spatial, un gradient intermédiaire (ZW) commuté avec le système d'IRM (50),
les gradients intermédiaires (ZW) étant dimensionnés de telle sorte que la particule (30) est maintenue sensiblement à la même position (M1, M2) en moyenne temporelle sur chaque bloc de mesure (MB1, MB2).

2. Procédé selon la revendication 1, **caractérisé en ce que**, pendant un bloc de mesure respectif (MB1, MB2), la particule (30) s'éloigne d'un point de départ (AP) pendant lesdites une ou plusieurs mesures individuelles (E1, E2), et que le gradient intermédiaire (ZW) est dimensionné de telle sorte que, pendant l'effet du gradient intermédiaire (ZW), la particule (30) revient approximativement au point de départ (AP).

3. Procédé selon la revendication 2, **caractérisé en ce que** la particule (30) se déplace pendant lesdites une ou plusieurs mesures individuelles (E1, E2) sous l'influence de la gravité (34) et/ou d'un écoulement (35) de la matrice liquide (31) et/ou d'un effet de la commutation de gradients à codage spatial.

4. Procédé selon l'une des revendications 1 ou 2, **caractérisé en ce que** la commutation de gradients à codage spatial est équilibrée, de sorte que, pendant une mesure individuelle (E1, E2), elle ne contribue pas, au total, à un changement de position de la particule (30).

5. Procédé selon l'une des revendications 1 à 3, **caractérisé en ce que** la commutation de gradients à codage spatial n'est pas équilibrée, de sorte qu'elle contribue à un changement de position de la particule (30) pendant une mesure individuelle (E1, E2), et **en ce que** les gradients intermédiaires (ZW) participent à compenser les contributions de la commutation de gradients à codage spatial.

6. Procédé selon l'une des revendications précédentes, **caractérisé en ce qu'**avant la séquence de mesure (MS1, MS2), en particulier avant l'introduction de la particule (30) dans la matrice liquide (31), une mesure des écoulements (33) dans la matrice liquide (31) est effectuée, par laquelle une contribution des écoulements (33) à un changement de position de la particule (30) peut être déterminée pendant une mesure individuelle (E1, E2) et/ou pendant une pause du codage spatial, et **en ce que** les gradients intermédiaires (ZW) participent à compenser les contributions des écoulements (33).

7. Procédé selon l'une des revendications précédentes, **caractérisé en ce que** le gradient intermédiaire (ZW) presse au moins temporairement la particule (30) contre une structure de bord (32) à laquelle la matrice liquide (31) est adjacente.

8. Procédé selon l'une des revendications précédentes, **caractérisé en ce qu'**une seule mesure individuelle (E1, E2) est effectuée par bloc de mesure (MB1, MB2).

9. Procédé selon l'une des revendications 1 à 8, **caractérisé en ce qu'**une image complète du volume de mesure (52) est générée à partir des résultats des mesures individuelles (E1, E2) de la séquence de mesure (MS1, MS2).

10. Procédé selon l'une des revendications 1 à 8, **caractérisé en ce qu'**avant la séquence de mesure (MS1, MS2), une pluralité de projections de référence du volume de mesure (52) sont réalisées sans la particule (30), en particulier par acquisition directe des projections de référence du volume de mesure (52) sans la particule (30) ou par calcul à partir d'une acquisition d'image complète du volume de mesure (52) sans la particule (30), **en ce que**, au moyen des mesures individuelles (E1, E2), une pluralité de projections du volume de mesure (52) avec la particule (30) est acquise, et **en ce que** la position de la particule (30) est déterminée en comparant les projections acquises avec les projections de référence.

11. Procédé de positionnement d'au moins une particule ferromagnétique (30) dans une matrice liquide (31) au moyen d'un système d'IRM (50), comprenant les étapes suivantes :
1) la position de la particule (30) est déterminée (S1) au moyen du système d'IRM (50) ;
2) un gradient de positionnement est commuté (23) avec le système d'IRM (50), avec lequel la position de la particule (30) est modifiée (S2),
**caractérisé en ce**
**que**, dans le cadre de l'étape 1), un procédé selon l'une des revendications 1 à 10 est mis en oeuvre,
dans lequel, après la fin de la séquence de mesure (20) à l'étape 1) (S1) jusqu'au début de l'application du gradient de positionnement (23) à l'étape 2) (S2), un gradient de fixation est commuté au moyen du système d'IRM (50), avec lequel la position de la particule (30) est maintenue sensiblement constante.

12. Procédé selon la revendication 11, **caractérisé en ce que** les étapes 1) (S1) et 2) (S2) sont répétées plusieurs fois.

13. Procédé selon la revendication 11 ou 12, **caractérisé en ce que**, sous l'effet du gradient de fixation, la particule (30) subit une force qui est opposée à l'effet de la gravité (34) et, le cas échéant, d'un écoulement (35) de la matrice liquide (31).

14. Procédé selon l'une des revendications 11 ou 12, **caractérisé en ce que** le gradient de fixation presse la particule (30) contre une structure de bord (32) à laquelle la matrice liquide (31) est adjacente.

15. Système d'IRM (50) comprenant un aimant (51) pour générer un champ magnétique homogène Bo dans un volume de mesure (52), un système de bobines de gradient (54) pour générer des gradients de champ magnétique à codage spatial dans le volume de mesure (52), et un système de bobines d'excitation et de lecture à haute fréquence (53) pour envoyer des impulsions à haute fréquence (1) dans le volume de mesure (52) et pour lire le volume de mesure (52),
**caractérisé en ce**
**que** le système d'IRM (50) est conçu pour déterminer la position d'une particule ferromagnétique (30) selon l'une des revendications 1 à 10, un moyen de commande (57) étant prévu, qui est configuré pour commuter des gradients intermédiaires (ZW) entre des mesures individuelles (E1, E2) d'une séquence de mesure (MS1, MS2) avec le système de bobines de gradient (54), et en ce que le système d'IRM (50) est en outre conçu pour positionner la particule ferromagnétique (30) selon l'une des revendications 11 à 14, le moyen de commande (57) étant configuré pour commuter des gradients de fixation entre la fin des séquences de mesure (20) et l'application de gradients de positionnement (23) avec le système de bobines de gradient (54).

16. Système d'IRM (50) selon la revendication 15, **caractérisé en ce que** le système de bobines de gradient (54) du système d'IRM (50) présente un premier sous-système de bobines (55) pour générer un gradient de champ magnétique dans une direction verticale (y) et au moins un second sous-système de bobines (56) pour générer un gradient de champ magnétique dans une direction horizontale (z, x),
et **en ce que** le premier sous-système de bobines (55) présente une force de gradient maximale produisible |Gₘₐₓ¹| qui est supérieure à une force de gradient maximale produisible |Gₘₐₓ²| du second sous-système de bobines (56), de préférence |Gₘₐₓ¹| ≥ 1,5 * |Gₘₐₓ²|.

17. Système d'IRM (50) selon la revendication 15 ou 16, **caractérisé en ce que** le système de bobines de gradient (54) du système d'IRM (50), en particulier le premier sous-système de bobines (55), comprend une partie principale (55a) et une partie supplémentaire (55b), le moyen de commande (57) étant conçu pour commuter des commutations de gradients à codage spatial, des gradients intermédiaires, des gradients de fixation et/ou des gradients de positionnement avec la partie principale (55a), et pour commuter uniquement des gradients intermédiaires, des gradients de fixation et/ou des gradients de positionnement, mais pas des commutations de gradients à codage spatial, avec la partie supplémentaire (55b).
